# EUROPEAN PATENT SPECIFICATION

(11) **EP 2 744 483 B1**
(45) Date of publication and mention of the grant of the patent: **29.04.2020**
(21) Application number: 12823626.2
(22) Date of filing: 08.08.2012
(51) Int. Cl.: A61K 9/16, A61K 9/14, A61K 48/00, A61K 31/7105, A61K 31/7088, A61P 35/00

(54) **DELIVERY SYSTEM FOR SPECIFICALLY TARGETING CANCER CELLS AND METHOD OF USE THEREOF**
FREISETZUNGSSYSTEM FÜR SPEZIFISCHES TARGETING VON KREBSZELLEN UND VERWENDUNGSVERFAHREN DAFÜR
SYSTÈME D'ADMINISTRATION POUR LE CIBLAGE SPÉCIFIQUE DE CELLULES CANCÉREUSES, ET SON PROCÉDÉ D'UTILISATION

(30) Priority: 15.08.2011 US 201161523626 P
(43) Date of publication of application: 25.06.2014
(73) Proprietor: Jyant Technologies, Inc., Marietta, GA 30008 (US)
(72) Inventor: LILLARD, James, W., Smyrna, GA 30082 (US)
(74) Representative: TL Brand & Co
(86) International application number: PCT/US2012/049988
(87) International publication number: WO 2013/025418

(56) References cited:
- US-A- 5 817 343
- US-A1- 2008 089 941
- US-A1- 2009 170 195
- US-A1- 2010 290 982
- US-A1- 2011 052 709
- THANGAPAZHAM RAJESH L ET AL: "Evaluation of a nanotechnology-based carrier for delivery of curcumin in prostate cancer cells", INTERNATIONAL JOURNAL OF ONCOLOGY, vol. 32, no. 5, May 2008 (2008-05), pages 1119-1123, XP055159528, ISSN: 1019-6439
- KIM T H ET AL: "Preparation and characterization of water-soluble albumin-bound curcumin nanoparticles with improved antitumor activity", INTERNATIONAL JOURNAL OF PHARMACEUTICS, ELSEVIER BV, NL, vol. 403, no. 1-2, 17 January 2011 (2011-01-17), pages 285-291, XP027554477, ISSN: 0378-5173 [retrieved on 2010-10-28]
- LIU SHUANGLIN ET AL: "Anti-tumor activity of curcumin against androgen-independent prostate cancer cells via inhibition of NF-[kappa]B and AP-1 pathway in vitro.", JOURNAL OF HUAZHONG UNIVERSITY OF SCIENCE AND TECHNOLOGY. MEDICAL SCIENCES = HUA ZHONG KE JI DA XUE XUE BAO. YI XUE YING DE WEN BAN = HUAZHONG KEJI DAXUE XUEBAO. YIXUE YINGDEWEN BAN AUG 2011, vol. 31, no. 4, 1 August 2011 (2011-08-01) , pages 530-534, XP019936046, ISSN: 1672-0733
- HONG J H ET AL: "The effects of curcumin on the invasiveness of prostate cancer in vitro and in vivo.", PROSTATE CANCER AND PROSTATIC DISEASES 2006, vol. 9, no. 2, 2006, pages 147-152, XP055159527, ISSN: 1365-7852
- GARCIA-BENNETT ALFONSO ET AL: "In search of the Holy Grail: Folate-targeted nanoparticles for cancer therapy.", BIOCHEMICAL PHARMACOLOGY 15 APR 2011, vol. 81, no. 8, 15 April 2011 (2011-04-15) , pages 976-984, XP055159522, ISSN: 1873-2968
- R. SINGH ET AL.: 'Nanoparticle-based targeted drug delivery' EXPERIMENTAL MOLCULAR PATHOLOGY vol. 86, no. 3, June 2009, pages 215 - 223, XP026096507

### SEQUENCE LISTING

<110> Morehouse School of Medicine
   Lillard, James W. Jr.
<120> Delivery System For Specifically Targeting Cancer Cells And Method of Use Thereof
<130> 1013-150 PCT
<150> 61/523,626
   <151> 2011-08-15
<160> 4
<170> PatentIn version 3.5
<210> 1
   <211> 12
   <212> PRT
   <213> Artificial Sequence
<220>
   <223> synthetic peptide
<400> 1
   His Thr Ile Arg Tyr Asp Trp His Phe Thr Ala Arg
   1               5                   10
<210> 2
   <211> 12
   <212> PRT
   <213> Artificial Sequence
<220>
   <223> synthetic peptide
<400> 2
   Phe Arg Pro Asn Arg Ala Gln Asp Tyr Asn Thr Asn
   1               5                   10
<210> 3
   <211> 12
   <212> PRT
   <213> Artificial Sequence
<220>
   <223> synthetic peptide
<400> 3
   Thr Ile His Tyr Asp Phe Trp His Thr Arg Arg Ala
   1               5                   10
<210> 4
   <211> 12
   <212> PRT
   <213> Artificial Sequence
<220>
   <223> synthetic peptide
<400> 4
   Asn Phe Thr Arg Pro Asn Asn Tyr Arg Asp Ala Gln
   1               5                   10

## Description

This application claims the priority of Provisional Application No. 61/523,626, filed August 15, 2011.

### FIELD

This application generally relates to a delivery system and treatment of cancer. In particular, the application relates to delivery of cytotoxic agents using nanoparticles.

### BACKGROUND

Systemic toxicity is a major problem associated with many cytotoxic drugs that disrupt a cell's ability to divide and replicate. For example, despite some of the benefit, platinum-based drugs (*e.g.,* carboplatin, cisplatin, oxaliplatin, satraplatin, triplatin tetranin, etc.), natural phenols (*e.g.,* cardamom, curcumin, galangal, ginger, melegueta pepper, turmeric, etc.), plant alkaloids and taxanes (*e.g.,* camptothecin, docetaxel, paclitaxel, vinblastine, vincristine, virorelbine, vincristine, etc.), other alkylating agents (*e.g.,* altretamine, busulfan, carmustine, chlorambucil, cyclophosphamide, dacarbazine, ethylenimines, haxmethyl melamine, hydrazines, ifosfamide, lomustine, mechlorethamine, melphalan, nitroosoureas, piperine, procarbazine, streptozocin, temozolomide, thiotepa, triazines, etc.), tumor antibiotics and anthracyclines (*e.g.,* bleomycin, chromomycin, dactinomycin, daunorubicin, doxorubicin, epirubicin, idarubicin, mitomycin, mitoxantrone, plicamycin, etc.), topoisomerase inhibitors (*e.g.,* amsacrine, etoposides, irinotecan, teniposides, toptecan, etc.), antimetabolites (5-fluorouracil, 6-thioguanine, 6-mercaptopurine, adenosine deaminase inhibtors, capecitabine, cladribine, cytarabine, foxuridine, fludarabine, gemcitabine, methotrexate, nelerabine, pentaostatin mitotic inhibitor, purine antagonists, pyrimidine antagonists, etc.), and miscellaneous antineoplastics (*e.g.,* asparaginase, bexarotene, estramustine, hydroxyurea, isotretinoin, mitotane, pegaspargase, retinoids, tretinoin, etc.) are higly efficient in kill cancer cells. However, their utility remains uncertain and fraut with complexities and problems. For example, platininum-based drugs are not the first treatment of choice for cancers, because these drugs are readily absorbed by healthy tissues and less than 1% of the administered drug make it to their intracellular target (*i.e*., cancer cell nuclei/DNA). As a result, platinum-based and other cancer drugs can be extremely toxic to heathy tissues. Therefore, there exists a need for a delivery system that specifically targets cancer cells for the deliver of cytotoxic agents.

Thangapazham Rajesh L et al.: "Evaluation of a nanotechnology-based carrier for delivery of curcumin in prostate cancer cells", International Journal of Oncology, 32(5), 2008, p1119 - 1123 discloses the use of liposomes coated with prostate specific antibodies comprising curcumin for the treatment of prostate cancer.

### SUMMARY

One aspect of the present application relates to a pharmaceutical composition according to Claim 1.

Also disclosed herein is a method for treatment of cancer in a subject in need thereof comprising, administering to the subject an effective amount of a system for specifically targeting cancer cells comprising high affinity and selective tumor cell ligand - coated planetary ball milled (PBM) nanoparticles containing a cytotoxic agent.

Also disclosed herein is a method of inhibiting cancer metastasis in a subject in need thereof comprising, administering to the subject an effective amount of a system for specifically targeting cancer cells comprising high affinity and selective tumor cell ligand - coated planetary ball milled (PBM) nanoparticles containing a cytotoxic agent.

Also disclosed herein is a method of inducing apoptosis of regulatory T (Treg) cells in a subject in need thereof comprising, administering to the subject an effective amount of high affinity and selective Treg ligand-coated planetary ball milled (PBM) nanoparticles containing an agent cytotoxic for Treg cells.

### BRIEF DESCRIPTION OF THE DRAWINGS

FIG. 1 shows an exemplary method for making biodegradable planetary ball-milled (PBM) nanoparticles.
FIG. 2 is a drawing of the molecular targets modulated by curcumin.
FIG. 3 shows expression of folate receptors by breast cancer tissue and cell lines.
FIG. 4 shows PBM nanoparticle-mediated apoptosis of breast cancer (BrCa) cells.
FIG. 5 shows PBM nanoparticle-mediated tumor regression in MDA-MB-231-Luc xenografts.
FIG. 6 shows PBM nanoparticle mediated apoptosis of CD4⁺CD25^{High}FoxP3⁺ (Treg) cells.
FIG. 7 shows that folate receptors (FRs) are expressed by both PC3 and PTEN-CaP2 tumors, but not by the cell lines or by normal cells.
FIG. 8 shows cisplatin-loaded folate-coated PBM nanoparticle mediated destruction of PC3 cells *in vitro.*
FIG. 9 shows tumor regression in PC3-luc tumor-bearing mice that received either cisplatin solution (8 mg/kg/week) or folate-coated XPclad nanoparticles containing cisplatin.
FIG. 10 shows patricle size distribution of coated and uncoated PBM nanoparticles.
FIG. 11 shows PBM nanoparticles coated with polycaprolactone with surface-conjugated folate.
FIG. 12 shows the zeta potential of prostate tumor specific PBM particles.
FIG. 13 shows expression of folate receptors by prostate cell lines and tumors.
FIG. 14 shows apoptosis of PC3 cells incubated with curcumin loaded PBM nanoparticles.
FIG. 15 shows an exemplary cartoon of the manufacture of PBM nanoparticles.
FIG. 16 shows mice injected intraventricularly and intratibialy with PC3 tumor cells.
FIG. 17 shows particle size distribution of prostate tumor-specific PBM nanoparticles.
FIG. 18 shows Zeta potential of prostate tumor-specific PBM nanoparticles.
FIG.19 shows Tunel and DRAQ5 staining of PBM nanoparticle-treated prostate cancer cells.
FIG. 20 shows PBM nanoparticle-mediated cancer and normal cell apoptosis.
FIG. 21 shows PBM nanoparticle translocation to cell nuclei and TUNEL stain.

### DETAILED DESCRIPTION

The following detailed description is presented to enable any person skilled in the art to make and use the invention. For purposes of explanation, specific nomenclature is set forth to provide a thorough understanding of the present invention. However, it will be apparent to one skilled in the art that these specific details are not required to practice the invention. Descriptions of specific applications are provided only as representative examples. The present invention is not intended to be limited to the embodiments shown, but is to be accorded the widest possible scope consistent with the principles and features disclosed herein.

Unless otherwise defined, scientific and technical terms used in connection with the present invention shall have the meanings that are commonly understood by those of ordinary skill in the art. Further, unless otherwise required by context, singular terms shall include pluralities and plural terms shall include the singular.

### Definitions

As used herein, the following terms shall have the following meanings:
The term "cancer" as used herein refers to a malignant neoplasm or malignant tumor and is a class of diseases in which a group of cells display uncontrolled growth, invasion that intrudes upon and destroys adjacent tissues, and sometimes metastasis, or spreading to other locations in the body via lymph or blood. These three malignant properties of cancers differentiate them from benign tumors, which do not invade or metastasize. The term "cancer" is inclusive here of "carcinoma," "sarcoma," "lymphoma," "leukemia," "melanoma," "germ cell tumor," and "blastoma."

The term "tumor" as used herein refers to a neoplasm or a solid lesion formed by an abnormal growth of cells. A tumor can be benign, pre-malignant or malignant.

The term "carcinoma" as used herein refers to an invasive malignant tumor consisting of transformed epithelial cells or transformed cells of unknown histogenesis, but which possess specific molecular or histological characteristics that are associated with epithelial cells, such as the production of cytokeratins or intercellular bridges. Exemplary carcinomas of the present invention include melanoma, ovarian cancer, vaginal cancer, cervical cancer, uterine cancer, prostate cancer, anal cancer, rectal cancer, colon cancer, stomach cancer, pancreatic cancer, insulinoma, adenocarcinoma, adenosquamous carcinoma, neuroendocrine tumor, breast cancer, lung cancer, esophageal cancer, oral cancer, brain cancer, medulloblastoma, neuroectodermal tumor, glioma, pituitary cancer, and bone cancer.

The term "sarcoma" as used herein refers to cancers arising from connective tissue, such as bone, cartilage, fat and nerve tissue, each of which develop from cells originating in mesenchymal cells outside the bone marrow, *i.e*., a cancer that arises from transformed cells in one of a number of tissues that develop from embryonic mesoderm. Thus, sarcomas include tumors of bone, cartilage, fat, muscle, vascular, and hematopoietic tissues. For example, osteosarcoma arises from bone, chondrosarcoma arises from cartilage, liposarcoma arises from fat, and leiomyosarcoma arises from smooth muscle. Exemplary sarcomas include: Askin's tumor, botryodies, chondrosarcoma, Ewing's-PNET, malignant Hemangioendothelioma, malignant Schwannoma, osteosarcoma, soft tissue sarcomas. Subclases of soft tissue sarcomas include: alveolar soft part sarcoma, angiosarcoma, cystosarcoma phyllodes, dermatofibrosarcoma, desmoid tumor, desmoplastic small round cell tumor, epithelioid sarcomaextraskeletal chondrosarcoma, extraskeletal osteosarcoma, fibrosarcoma, hemangiopericytoma, hemangiosarcoma, Kaposi's sarcoma, leiomyosarcoma, liposarcoma, lymphangiosarcomal, lymphosarcoma, malignant fibrous histiocytoma, neurofibrosarcoma, rhabdomyosarcoma, and synovial sarcoma.

The term "lymphoma" as used herein refers to cancers of lymphatic cells of the immune system arising from hematopoietic cells that leave the marrow and mature in the lymph nodes. Lymphomas typically present as a solid tumor. Exemplary lymphomas include: small lymphocytic lymphoma, lymphoplasmacytic lymphoma, Waldenstrom macroglobulinemia, splenic marginal zone lymphoma, plasmacytoma, extranodal marginal zone B cell lymphoma, MALT lymphoma, nodal marginal zone B cell lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, Burkitt lymphoma, B cell chronic lymphocytic lymphoma, classical Hodgkin lymphoma, nodular lymphocyte-predominant Hodgkin lymphoma, adult T cell lymphoma, nasal type extranodal NK/T cell lymphoma, enteropathy-type T cell lymphoma, hepatosplenic T cell lymphoma, blastic NK cell lymphoma, mycosis fungoide, Sezary syndrome, primary cutaneous CD30-positive T cell lympho-proliferative disorders, primary cutaneous anaplastic large cell lymphoma, lymphomatoid papulosis, angioimmunoblastic T cell lymphoma, unspecified peripheral T cell lymphoma, and anaplastic large cell lymphoma. Exemplary forms of classical Hodgkin lymphoma including: nodular sclerosis, mixed cellularity, lymphocyte-rich, and lymphocyte-depleted or not depleted.

The term "leukemia" as used herein refers to cancers arising from hematopoietic cells that leave the marrow and mature in the blood. Leukemia is a broad term covering a spectrum of diseases. In turn, it is part of the even broader group of diseases called hematological neoplasms. Leukemia is subdivided into a variety of large groups; the first division is between acute and chronic forms of leukemia. Acute leukemia is characterized by a rapid increase in the numbers of immature blood cells. Crowding due to such cells makes the bone marrow unable to produce healthy blood cells. Chronic leukemia is characterized by the excessive build up of relatively mature, but still abnormal, white blood cells. Typically taking months or years to progress, the cells are produced at a much higher rate than normal cells, resulting in many abnormal white blood cells in the blood. Leukemia is also subdivided by the blood cells affected. This split divides leukemias into lymphoblastic or lymphocytic leukemias and myeloid or myelogenous leukemias. In lymphoblastic or lymphocytic leukemias, the cancerous change takes place in a type of marrow cell that normally goes on to form lymphocytes. In myeloid or myelogenous leukemias, the cancerous change takes place in a type of marrow cell that normally goes on to form red blood cells, some other types of white cells, and platelets. Combining these two classifications provides a total of four main categories. Within each of these four main categories, there are typically several subcategories. There are also rare types outside of this classification scheme. Exemplary leukemias include: acute lymphoblastic leukemia (ALL), chronic lymphocytic leukemia (CLL), acute myelogenous leukemia (AML), chronic myelogenous leukemia (CML), hairy cell leukemia (HCL), T-cell prolymphocytic leukemia, large granular lymphocytic leukemia, juvenile myelomonocytic leukemia, B-cell prolymphocytic leukemia, Burkitt leukemia, and adult T-cell leukemia.

The term "melanoma" as used herein is a cancer or malignant tumor of melanocytes. Melanocytes are cells that produce the dark pigment, melanin, which is responsible for the color of skin. They predominantly occur in skin, but are also found in other parts of the body, including the bowel and the eye. Melanoma is divided into the following stereotypes and subtypes: lentigo maligna, lentigo maligna melanoma, superficial spreading melanoma, acral lentiginous melanoma, mucosal melanoma, nodular melanoma, polypoid melanoma, desmoplastic melanoma, amelanotic melanoma, soft-tissue melanoma, melanoma with small nevus-like cells, melanoma with features of a Spitz nevus, and uveal melanoma.

The term "germ cell tumor" as used herein refers to cancers derived from germ or pluripotent cells, including those presenting in the testicle (seminoma) or the ovary (dysgerminoma). Germ cell tumors can be cancerous or non-cancerous tumors. Germ cells normally occur inside the gonads (ovary and testis). Germ cell tumors that originate outside the gonads may be birth defects resulting from errors during development of the embryo. Germ cell tumors are broadly divided in two classes: germinomatous or seminomatous and nongerminomatous or nonseminomatous germ cell tumors. Exemplary germinomatous or seminomatous germ cell tumors include: germinoma, dysgerminoma, and seminoma. Exemplary nongerminomatous or nonseminomatous germ cell tumors include: Embryonal carcinoma, endodermal sinus tumor or yolk sac tumor (EST, YST), choriocarcinoma, mature teratoma, dermoid cyst, immature teratoma, teratoma with malignant transformation, polyembryoma, gonadoblastoma, and mixed GCT.

The term "blastoma" as used herein refers to cancers derived from immature precursor cells or embryonic tissue.

The term "BrCa" as used herein refers to breast cancer. The term "PCa" as used herein refers to prostate cancer.

The term "metastasis" as used herein refers to the spread of a cancer or carcinoma from one organ or part to another non-adjacent organ or part.

As used herein, the term "antibody" refers to immunoglobulin molecules and immunologically active portions of immunoglobulin (Ig) molecules, *i.e.,* molecules that contain an antigen binding site that specifically binds (immunoreacts with) an antigen. The term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity. By "specifically bind" or "immunoreacts with" is meant that the antibody reacts with one or more antigenic determinants of the desired antigen and does not react (*i.e*., bind) with other polypeptides or binds at much lower affinity with other polypeptides. The term "antibody" also includes antibody fragments that comprise a portion of a full length antibody, generally the antigen binding or variable region thereof. Examples of antibody fragments include Fab, Fab', F(ab')2, and Fv fragments; diabodies; linear antibodies; single-chain antibody (scFv) molecules; and multispecific antibodies formed from antibody fragments. In certain embodiments of the invention, it may be desirable to use an antibody fragment, rather than an intact antibody, to increase tumor penetration, for example. In this case, it may be desirable to use an antibody fragment that has been modified by any means known in the art in order to increase its serum half life.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity.

"Humanized" forms of non-human antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and/or capacity. Methods for making humanized and other chimeric antibodies are known in the art.

"Bispecific antibodies" are antibodies that have binding specificities for at least two different antigens. Methods for making bispecific antibodies are known in the art.

The use of "heteroconjugate antibodies" is also within the scope of the present invention. Heteroconjugate antibodies are composed of two covalently joined antibodies. Such antibodies have, for example, been proposed to target immune system cells to unwanted cells (U.S. Patent No. 4,676,980). It is contemplated that the antibodies can be prepared in vitro using known methods in synthetic protein chemistry, including those involving crosslinking agents.

The present application also contemplates the use of "immunoconjugates" comprising an antibody conjugated to a cytotoxic agent such as a toxin (e.g., an enzymatically active toxin of bacterial, fungal, plant, or animal origin, or fragments thereof), or a radioactive isotope (*i.e.,* a radioconjugate). Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include 212Bi, 1311, 131In, 90Y, and 186Re.

"Mammal" for purposes of treatment refers to any animal classified as a mammal, including humans, non-human primates, domestic and farm animals, and zoo, sports, or pet animals, such as dogs, horses, cats, cows, *etc.* Preferably, the mammal is human.

The terms "treat," "treating" or "treatment" as used herein, refers to a method of alleviating or abrogating a disorder and/or its attendant symptoms. The terms "prevent", "preventing" or "prevention," as used herein, refer to a method of barring a subject from acquiring a disorder and/or its attendant symptoms. In certain embodiments, the terms "prevent," "preventing" or "prevention" refer to a method of reducing the risk of acquiring a disorder and/or its attendant symptoms.

The term "inhibits" is a relative term, an agent inhibits a response or condition if the response or condition is quantitatively diminished following administration of the agent, or if it is diminished following administration of the agent, as compared to a reference agent. Similarly, the term "prevents" does not necessarily mean that an agent completely eliminates the response or condition, so long as at least one characteristic of the response or condition is eliminated. Thus, a composition that reduces or prevents tumor growth or a response, such as a pathological response, can, but does not necessarily completely eliminate such growth or response, so long as the growth or response is measurably diminished, for example, by at least about 50%, such as by at least about 70%, or about 80%, or even by about 90% of (that is to 10% or less than) the growth or response in the absence of the agent, or in comparison to a reference agent.

The term "increased level" refers to a level that is higher than a normal or control level customarily defined or used in the relevant art. For example, an increased level of immunostaining in a tissue is a level of immunostaining that would be considered higher than the level of immunostaining in a control tissue by a person of ordinary skill in the art.

The term "biological sample," as used herein, refers to material of a biological origin, which may be a body fluid or body product such as blood, plasma, urine, saliva, spinal fluid, stool, sweat or breath. Biological sample also includes tissue samples and cell samples.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10" as well as "greater than or equal to 10" is also disclosed.

In the context of the present application, an "effective amount" refers to the amount of a composition sufficient to effect beneficial or desired results, e.g., an amount effective in the prevention or treatment of a disorder. A "disorder" is any condition that would benefit from treatment with the antibody, including carcinoma and chemoresistance. This includes chronic and acute disorders or diseases including those pathological conditions, which predispose the mammal to the disorder in question.

### Drug-Carrying Nanoparticles That Specifically Target Cancer Cells

One aspect of the present application relates to drug-carrying nanoparticles that specifially target certain cells, such as cancer cells and regulatory T cells (Treg cells). The drug-carrying nanoparticles comprise a planetary ball milled nanoparticle core, one or more cytotoxic agents, and one or more cell targeting agents. In certain embodiments, the one or more cytotoxic agents comprise curcumin and the one or more cell targeting agents comprise folate. In certain other embodiments, the one or more cytotoxic agents comprise curcumin and one or more other cytotoxic agents. The drug-carrying nanoparticles of the present application allow for the use of a lower dose of cytotoxic drugs, reduce adverse events, increase efficacy, and reduce the possibility of the drugs being rapidly cleared from targeted tumor or cancer cells. For example, the drug-carrying nanoparticles of the present application are able to target and be taken up by breast or prostate cancer cells, thus would effectively deliver chemotherapeutic agents to intracellular targets in the cancer cells and carry sufficient drug to complete the process of apoptosis to limit the potential of chemoresistance and systemic toxicities.

### Planetary Ball Milled (PBM) Nanoparticles

As used herein, the terms "PBM nanoparticle" and "XPclad nanoparticle" are equivalent and may be used interchangeably. Insome embodiments, the PBM nanoparticles have diameters in the range of 0.1 nm - 60 µm, 0.1 nm - 1 µm, 0.1 nm - 500 nm, 0.1 nm - 200 nm, 1 nm - 200 nm, 5 nm - 60 µm, 5 nm - 30 nm, 30 nm - 80 nm, 30 nm - 180 nm, 80 nm - 1 µm, 180 nm - 1 µm, 200 nm - 1 µm, 1 µm - 6 µm, 4 µm - 12 µm, and 10 µm to 60 µm. In other embodiments, the PBM nanoparticles are biodegradable nanoparticles. The PBM nanoparticles can be made using a novel formulation method to generate particles of uniform size, 100% loading efficiency of hydrophobic or hydrophilic drugs, subsequent coating for targeted delivery, and control of surface logP (metric for lipophilic:hydrophilic distribution) for systemic, oral, or cutaneous delivery.

The PBM nanoparticles as described in the present application represents a novel nanoparticle formulation method that uses planetary ball milling to generate particles of uniform size, 100% loading efficiency of hydrophobic or hydrophilic drugs, subsequent coating for targeted delivery, and control of surface logP for systemic, oral, or cutaneous delivery. These are the first studies of PBM nanoparticles whereby uniform-sized particle with engineered surface characteristics target FR1 on breast tumors and FR4-expressing Treg cells without clearance by the RES.

In some embodiments, the PBM nanoparticle comprise a nano-matrix core comprising one or more biodegradable polymer or polysaccharide such as alginate, cellulose, collagen and starch. Other suitable biodegradable polymers include, but are not limited to, polyethylene glycol (PEG), polyglycolic acid (PGA), polylactic acid (PLA), lactic acid-glycolic acid copolymer (PLGA), polyhydroxyalkanoates (PHA), polyhydroxybutyrate-valerate (PHBV), polyvinyl alcohol (PVA), polyethylene terephthalate (PET), polyglycolide-lactide, polycaprolactone (PCL), lactic acid-ε-caprolactone copolymer (PLCL), polydioxanone (PDO), polytrimethylene carbonate (PTMC), poly(amino acid), polydioxanone, polyoxalate, a polyanhydride, a poly(phosphoester), polyorthoester and copolymers thereof. In certain embodiments, the nanoparticle core matrix comprises a mixture of PEG and a polymer or polysaccharide selected from the group consisting of alginate, cellulose, collagen and starch. Molecules of the cytotoxic agent are entrapped in the nanoparticle core matrix. In some embodiments, the cytotoxic agents are mixed with the biodegradable polymers and polysaccharides during the formation of the matrix core. In other embodiemtns, the matrix core is formed first and the cytotoxic agent(s) are loaded at a later stage.

In some embodiments, the matrix core is formed by dissolving the biopolymer, such as alginate, cellulose, collagen and starch, in water to form an aqueous solution, adding the cytotoxic agent to the squeous solution to form a biopolymer/cytotoxic agent mixture and, optionally, adding another polymer, such as PEG, to the biopolymer/cytotoxic agent mixture to form a final mixture. The final mixture is dried into pellet or tablet form and then milled using planetary ball milling under controlled temperature (<37°C). The size of the nanoparticles may be controled by speed and duration of the planetary ball milling. In some embodiments, the nanoparticles have a size range of 0.1 to 5 nm, 5 to 30 nm, 30 to 80 nm, 30 to 180 nm, 180 to 1000 nm or 200 to 1000 nm. The grinding speed is in the range of 100-600 rpm, preferably 200-400 rpm.

In other embodiments, the PBM nanoparticles are further coated with a release control polymer coating to control the time of release of contents, increase mechanical strength of the particles, and/or stabilize the activie ingredient(s) in the PBM nanoparticles. Polymers suitable for the release control coating include, but are not limited to, polycarprolactone (PCL) and PEG. In other embodiments, the PBM nanoparticles may be further coated, conjugated to or modified with a tumor-specific or cell/tissue specific targeting agent. Examples of the tumor-specific or cell/tissue specific targeting agents in clude, but are not limited to, lysine, peptide, antibodies and folate.

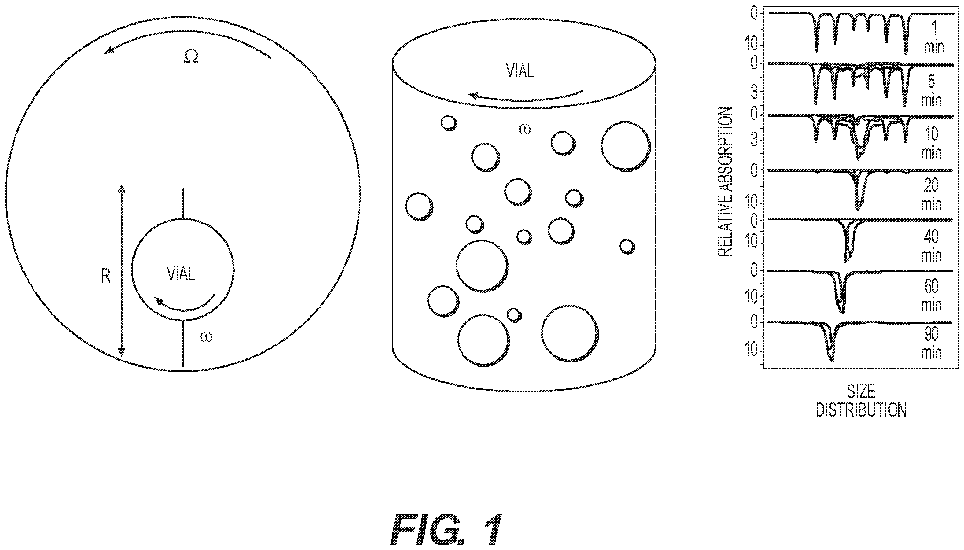

Biodegradable planetary ball-milled (PBM) nanoparticles, as exemplified in FIG. 1, have increased serum half-lives of encapsulated drug due to PEG-coating and having a negative LogP value.

There are currently several types of nanocarriers for drug delivery: polymeric, micelles, dendrimers, liposomes, albumin, viral, carbon nanotubes, and silica/metallic. While these nanoparticles have a number of limitations (*i.e*., nonuniform size, difficult to manufacture and/or lack of targeting) exist that reduces their utility.

In some embodiments, PBM nanoparticles formulated for oral delivery by coating with oral anticancer agents including, but not limited to, satraplatin, capecitabine, or erlotinib. Nanoparticles for oral delivery modulate surface LogP values for intestinal uptake and the addition of copolymers that are hydrolyzed during the first pass effect, transit through the hepatic biliary system without liver toxicity, and targeted-systemic delivery to local or metastatic tumors, including breast and prostate tumors. Formulations can also be made to include substrates for detection of micro-metastases and activated treatment.

### Cytotoxic Agents

A wide variety of cytotoxic agents with different intracellular targets can induce the uniform phenotype of apoptosis. This means that the cytotoxic activity of cytotoxic drugs is not solely dependent on specific drug-target interaction, but also on the activity of apoptotic (cell signaling) machinery of the cancer cell. Examples of cytotoxic agents include, but are not limited to, platinum-based drugs (*e.g.,* carboplatin, cisplatin, oxaliplatin, satrap latin, triplatin tetranin, and carboplatin *etc.*), natural phenols (*e.g.,* cardamom, curcumin, galangal, ginger, melegueta pepper, turmeric, etc.), plant alkaloids and taxanes (*e.g.,* camptothecin, docetaxel, paclitaxel, vinblastine, vincristine, virorelbine, vincristine, *etc.*), other alkylating agents (*e.g.,* altretamine, busulfan, carmustine, chlorambucil, cyclophosphamide, dacarbazine, ethylenimines, haxmethyl melamine, hydrazines, ifosfamide, lomustine, mechlorethamine, melphalan, nitroosoureas, piperine, procarbazine, streptozocin, temozolomide, thiotepa, triazines, *etc.),* tumor antibiotics and anthracyclines (*e.g*., bleomycin, chromomycin, dactinomycin, daunorubicin, doxorubicin, epirubicin, idarubicin, mitomycin, mitoxantrone, plicamycin, *etc.*), topoisomerase inhibitors (*e.g.,* amsacrine, etoposides, irinotecan, teniposides, toptecan, *etc.*), antimetabolites (*e.g.,* 5-fluorouracil, 6-thioguanine, 6-mercaptopurine, adenosine deaminase inhibtors, capecitabine, cladribine, cytarabine, foxuridine, fludarabine, gemcitabine, methotrexate, nelerabine, pentaostatin mitotic inhibitor, purine antagonists, pyrimidine antagonists, *etc.*), and miscellaneous antineoplastics (*e.g.*, asparaginase, bexarotene, estramustine, hydroxyurea, isotretinoin, mitotane, pegaspargase, retinoids, tretinoin, *etc*.)

Platinum-based therapies are widely used and function by damaging DNA and inducing apoptosis by cytochrome C release and subsequent caspase activation. Many chemoresistant cell lines are more sensitive to platinum-baseddrugs such as docetaxel and carboplatin. However, these drugs have many side effects including, but not limited to: neutropenia, liver dysfunction, anemia (70%), nausea (40%), infections (20%), neuromotor dysfunction (15%), renal failure and possibly cardiotoxicity - contributing to 1.7% of patient deaths receiving this therapy. These side effects underscore the need for innovative approaches that overcome or circumvent intrinsic mechanisms of drug efficacy, resistance, toxicity as well as simultaneously impede early stage primary or advance (*e.g.,* metastatic, triple negative) disease.

Despite extensive clinical experience with docetaxel, there is still a high level of unpredictability in regards to its efficacy and toxicity that can limit its use. Docetaxel is metabolized by CYP3-mediated oxidation, which results in metabolites with reduced cytotoxicity. This elimination pathway is dictated in part by the drug efflux transporter, ABCB1, but the C1236T polymorphism in the *ABCB1* gene significantly decreases docetaxel clearance. There is also evidence that polymorphisms in genes coding for DNA repair (*e.g., NER* and *BER*) and metabolic inactivation enzymes might contribute to individual differences in anti-tumor efficacy as well as toxicity of carboplatin. Several mechanisms are described that confer toxicity, decreased sensitivity, and resistance to carboplatin which include efflux pumps (*e.g., ABCB1*), increased detoxification enzymes, and associated *NER* and *BER,* respectively. In the context of platinum-based therapies, these polymorphisms can reduce *NER*/*BER* functionality and repair of platinum-DNA lesions.

In the present invention, the cytotoxic agent comprises curcumin. In an embodiment, the cytotoxic agent comprises curcumin and one or more other cytotoxic agents.

In another particular embodiment, the other cytotoxic agent is selected from the group consisting of a chemotherapeutic agent, radioconjugate, antibody, siRNA, antisense, triplex-forming oligonucleotide, and any other agent that is cytotoxic to cancer cells.

In a further embodiment, the other chemotherapeutic agent is selected from the group consisting of platinum-based drugs, natural phenols, plant alkaloids and taxanes, other alkylating agents, tumor antibiotics and anthracyclines, topoisomerase inhibitors, antimetabolites, and miscellaneous antineoplastics .

In another further embodiment, the radioconjugate comprises a radioisotope selected from the group consisting of ²¹²Bi, ¹³¹I, ¹³¹In, ⁹⁰Y, ¹⁸⁸Tu and ¹⁸⁶Re.

In another further embodiment, the antibody is capable of binding a tumor associated antigens selected from the group consisting of adenosine receptors, αVβ3 integrin, aminopeptidase P, alphaefetoprotein, cancer antigen 125, carcinoembryonic antigen, caveolin-1, chemokine receptors, clusterin, oncofetal antigens, CD20, epithelial tumor antigen, melanoma associated antigen, Ras, p53, Her2/Neu, ErbB2, ErbB3, ErbB4, folate receptor, prostate-specific membrane antigen, prostate specific antigen, purine receptors, radiation-induced cell surface receptor, serpin B3, serpin B4, squamous cell carcinoma antigens, thrombospondin, tumor antigen 4, tumor-associated glycoprotein 72, tyosinase and tyrosine kinases.

Other agents that are cytotoxic to cancer cells include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, a tricothecene, and fragments thereof.

### Curcumin

Curcumin (diferuloylmethane) is a polyphenol-derived from the plant *Curcuma longa,* commonly called turmeric. This is well-known hydrophobic, natural compound holds a high place in ayurvedic medicine as a "cleanser of the body" with antioxidant, anti-inflammatory, anti-carcinogenic, anti-proliferative and anti-angiogenic properties. But curcumin's hydrophobicity results in poor bioavailability, which hampers the efficacy of curcumin and its efficient role as anticancer agent. Therefore, the key challenges are the delivery of the curcumin directly to the cancer cells to increase its effectiveness and induce cancer cell death, while preventing damage to the normal cells.

Long circulating nanoparticles that are able to overcome the poor bioavailability of curcumin and avoid clearance by the reticulo-endothelial system (RES) would result in accumulation by tumors that have increased permeability and defective lymphatic drainage (*i.e*., enhance permeability and retention-EPR effect). Uptake of these nano-sized vehicles would have several advantages, including significantly reduced systemic toxicity due to targeted delivery. It is also plausible that these nanoparticles would prevent the occurrence of chemoresistance. Nanoparticles able to target and be taken up by prostate tumors would effectively deliver curcumin to perturb intracellular targets and sufficiently bioavailable to induce the process of apoptosis in cancer cells.

### Release Control Polymer Coating

The release control polymer coating controls the time of release of contents, increase mechanical strength of the coated PBM nanoparticles, and/or stabilize the activie ingredient(s) in the PBM nanoparticles. Polymers suitable for the release control coating include, but are not limited to, PEG, polycarprolactone (PCL), chaemically modified PCL and mixtures thereof. In some embodiments, the release control polymer coated PBM nanoparticles are further coated, conjugated to or modified with a cell targeting agent to specifically target, for example, cancer cells or Treg cells.

### Cell Targeting Agents

The cell targeting agent allow the drug-containing nanoparticles to target the specific types of cells of interest. The cell targeting agents are folate, adenosine, purine, hormone or peptide ligand that bind to or target a tumor associated antigen. Examples of tumor associated antigens include, but are not limited to, adenosine receptors, alpha v beta 3, aminopeptidase P, alphaefetoprotein, cancer antigen 125, carcinoembryonic antigen, cCaveolin-1, chemokine receptors, clusterin, oncofetal antigens, CD20, epithelial tumor antigen, melanoma associated antigen, Ras, p53, Her2/Neu, ErbB2, ErbB3, ErbB4, folate receptor, prostate-specific membrane antigen, prostate specific antigen, purine receptors, radiation-induced cell surface receptor, serpin B3, serpin B4, squamous cell carcinoma antigens, thrombospondin, tumor antigen 4, tumor-associated glycoprotein 72, tyosinase, and tyrosine kinases. In some embodiments, the cell targeting agent is folate or a folate derivative that binds specifically to folate receptors (FRs).

The reduced folate carrier (RFC) is a low-affinity, high capacity system that mediates the uptake of reduced folates into cancer cells at pharmacologic (µM) concentrations. The concentration of physiologic folates is in the range of 5 to 50 nM. Therefore, high affinity human FRs exist and are encoded by a family of genes whose homologous products are termed FR type α, β, γ, or δ, which are also described as FR1, FR2, FR3, or FR4, respectively. The membrane isoforms FR1, FR2, and FR4 can bind and transport folate or folate derivatives into the cell, while FR3 lacks a membrane anchor and is secreted from the cell. FR1 and FR2 bind folate and 6S 5-formyltetrahydrofolate (*i.e.,* leucovorin) with similar yet different affinities 1.5 nM *versus* 0.35 nM (folate) and 800 nM *versus* 7 nM (leucovorin), respectively. 6S 5-methyltetrahydrofolate is the predominate folate in the blood and has similar affinities for FR1 and FR2, 55 nM and 1 nM, respectively. While PC3 human prostate cancer cells do not significantly express FR (*e.g.,* FR1) in culture, FRs are expressed by PC3 tumors. FRs are also expressed by BrCa cells are associated with poor outcomes or transport folate via these receptors despite resistance to methotrexate.

Most nonproliferative tissues lack functional FR expression. FR expression in proliferating normal tissues is restricted to the luminal surface of certain epithelial cells and thus inaccessible to the circulation. However, the presence of high levels of FR2 (high affinity receptor) on malignant tumors and leukemias are exposed to circulation making them an attractive candidate for tumor-specific therapeutics. The kidney, where FR1 (moderate affinity receptor) is expressed in the proximal tubules, is protected from FR-targeted therapies that are excluded from glomerular filtration. Further protection is a result of the renal folate conservation mechanism where after FR-mediated endocytosis by renal tubular cells there is rapid dissociation of the folate and transport across the basolateral membranes into the blood.

Methotrexate resistance of cancer cells is largely due to alterations in the dihydrofolate reductase gene, reduced intracellular polyglutamation, increased efflux and transport via the RFC; thus, resistance to the biodegradable planetary ball milled (PBM) nanoparticles of the present application is unlikely since this therapy acts via FR binding and transport, which are only modestly affected in methotrexate resistant tumors.

The use of folate as a targeting agent in the PBM nanoparticles also allow both tumor cells and regulatory T (Treg) cells. It is well accepted that high numbers of Treg cells suppress tumor immunity. Specifically, T reg cells suppress (foreign and self) reactive T cells without killing them through contact-dependent or cytokine (*e.g.,* IL-10, TGF-β, *etc*.) secretion. FR4 is selectively upregulated on Treg cells. It has been shown that antibody blockade of FR4 depleted Treg cells and provoked tumor immunity in tumor-bearing mice. Thus, folate-coated PBM nanonparticles carrying a cytotoxic agent would take FR-expressing cells for their destruction, which would both directly (*i.e*., BrCa cell) and indirectly (*i.e*., breast tumor associated and peripheral Treg cells) inhibit tumor progression.

In another further embodiment, the targeting agent is an antibody or peptide capable of binding tumor associated antigens consisting of put not limited to: adenosine receptors, alpha v beta 3, aminopeptidase P, alphaefetoprotein, cancer antigen 125, carcinoembryonic antigen, caveolin-1, chemokine receptors, clusterin, oncofetal antigens, CD20, epithelial tumor antigen, melanoma associated antigen, Ras, p53, Her2/Neu, ErbB2, ErbB3, ErbB4, folate receptor, prostate-specific membrane antigen, prostate specific antigen, purine receptors, radiation-induced cell surface receptor, serpin B3, serpin B4, squamous cell carcinoma antigens, thrombospondin, tumor antigen 4, tumor-associated glycoprotein 72, tyosinase, tyrosine kinases, etc.

### TREATMENT METHODS

Also disclosed herein is a method for treatment of cancer in a subject in need thereof comprising, administering to the subject an effective amount of a pharmacutical composition comprising planetary ball milled (PBM) nanoparticles containing a cytotoxic agent and a cell targeting agent. The cell targeting agent may be folate or a folate derivative.
the drug-containing PBM nanoparticles are long circulating nanoparticles that are able to resist phagocytosis by macrophages and avoid clearance by the reticuloendothelial system (RES) which, in turn, would result in accumulation by tumors that have increased permeability and defective lymphatic drainage (*i.e*., enhance permeability and retention - EPR effect). Uptake of these nano-sized vehicles would have several benefits. The drug-containing PBM nanoparticles of the present application significantly reduced systemic toxicity due to targeted delivery.

Drug-containing PBM nanoparticles of the present application may prevent the occurrence of chemoresistance. Drug resistance is mediated through two broad mechanisms: (i) failure of sufficient amount of cytotoxic drug to reach the target cell (and intracellular site of action) and (ii) failure to complete cell death following induction of apoptotic mechanisms. Nanoparticles able to target and be taken up by breast tumors would effectively deliver chemotherapies as well as intracellular targets and carry sufficient drug to complete the process of apoptosis.

The cancer may be selected from the group consisting of carcinoma, sarcoma, lymphoma, leukemia, germ cell tumor, and blastoma.

The carcinoma may be prostate cancer. The cancer may be prostate cancer and the pharmacutical composition of the present alication may be administered in conjunction with an effective amount of vitamin D. Extremely low vitamin D status of some men has also been indicated in PCa progression. Moreover, cancer drug-related hypocalcemia is usually mild, but may be severe if patients have pre-existing vitamin D deficiencies. Vitamin D can effectively protect against the renal toxicity due to platinum-based cancer therapies and regulates NER gene targets to modulate DNA repair, which contributes to PCa progression and drug response.

The carcinoma may be breast cancer.

The cytotoxic agent may be curcumin.

The cytotoxic agent may be selected from the group consisting of a chemotherapeutic agent, radioconjugate, antibody, siRNA, antisense, triplex-forming oligonucleotide, and any other agent that is cytotoxic to cancer cells.

The chemotherapeutic agent may be selected from the group consisting of platinum-based drugs, natural phenols, plant alkaloids and taxanes, other alkylating agents, tumor antibiotics and anthracyclines, topoisomerase inhibitors, antimetabolites, and miscellaneous antineoplastics.

The radioconjugate may comprise a radioisotope selected from the group consisting of ²¹²Bi, ¹³¹I, ¹³¹In, ⁹⁰Y, ¹⁸⁸Tu, and ¹⁸⁶Re.

The antibody or peptide capable of binding tumor associated antigens may consist of but is not limited to: adenosine receptors, alpha v beta 3, aminopeptidase P, alphaefetoprotein, cancer antigen 125, carcinoembryonic antigen, caveolin-1, chemokine receptors, clusterin, oncofetal antigens, CD20, epithelial tumor antigen, melanoma associated antigen, Ras, p53, Her2/Neu, ErbB2, ErbB3, ErbB4, folate receptor, prostate-specific membrane antigen, prostate specific antigen, purine receptors, radiation-induced cell surface receptor, serpin B3, serpin B4, squamous cell carcinoma antigens, thrombospondin, tumor antigen 4, tumor-associated glycoprotein 72, tyosinase, tyrosine kinases, etc..

The agent that is cytotoxic to cancer cells may be selected from the group consisting of diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, a tricothecene, and fragments thereof.

The cytotoxic agent may comprise natural phenol and one or more other other cytotoxic agent selected from the group consisting of a chemotherapeutic agent, radioconjugate, antibody, peptide, siRNA, antisense, triplex-forming oligonucleotide, and any other agent that is cytotoxic to cancer cells.

Also disclosed herein is a method of inhibiting cancer metastasis in a subject in need thereof comprising: administering to the subject an effective amount of a pharmacutical composition comprising planetary ball milled (PBM) nanoparticles containing a cytotoxic agent and a cell targeting agent. The cell-targeting agent may be folate or a folate derivative.

The cancer may be selected from the group consisting of carcinoma, sarcoma, lymphoma, leukemia, germ cell tumor, and blastoma.

The carcinoma may be prostate cancer.

The carcinoma may be breast cancer.

The cytotoxic agent may be curcumin.

The cytotoxic agent may be selected from the group consisting of a chemotherapeutic agent, radioconjugate, antibody, siRNA, antisense, triplex-forming oligonucleotide, and any other agent that is cytotoxic to cancer cells.

The chemotherapeutic agent may be selected from the group consisting of platinum-based drugs, natural phenols, plant alkaloids and taxanes, other alkylating agents, tumor antibiotics and anthracyclines, topoisomerase inhibitors, antimetabolites, and miscellaneous antineoplastics.

The radioconjugate may comprise a radioisotope selected from the group consisting of ²¹²Bi, ¹³¹I, ¹³¹In, ⁹⁰Y, ¹⁸⁸Tu, and ¹⁸⁶Re.

The antibody or peptide capable of binding tumor associated antigens may consist of but is not limited to: adenosine receptors, alpha v beta 3, aminopeptidase P, alphaefetoprotein, cancer antigen 125, carcinoembryonic antigen, caveolin-1, chemokine receptors, clusterin, oncofetal antigens, CD20, epithelial tumor antigen, melanoma associated antigen, Ras, p53, Her2/Neu, ErbB2, ErbB3, ErbB4, folate receptor, prostate-specific membrane antigen, prostate specific antigen, purine receptors, radiation-induced cell surface receptor, serpin B3, serpin B4, squamous cell carcinoma antigens, thrombospondin, tumor antigen 4, tumor-associated glycoprotein 72, tyosinase, tyrosine kinases, etc.

The agent that is cytotoxic to cancer cells may be selected from the group consisting of diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, a tricothecene, and fragments thereof.

The cytotoxic agent may comprise curcumin and one or more other other cytotoxic agent selected from the group consisting of a chemotherapeutic agent, radioconjugate, antibody, siRNA, antisense, triplex-forming oligonucleotide, and any other agent that is cytotoxic to cancer cells.

The targeting ligand may comprise antibody or peptide capable of binding tumor associated antigens consisting of put not limited to: adenosine receptors, alpha v beta 3, aminopeptidase P, alphaefetoprotein, cancer antigen 125, carcinoembryonic antigen, caveolin-1, chemokine receptors, clusterin, oncofetal antigens, CD20, epithelial tumor antigen, melanoma associated antigen, Ras, p53, Her2/Neu, ErbB2, ErbB3, ErbB4, folate receptor, prostate-specific membrane antigen, prostate specific antigen, purine receptors, radiation-induced cell surface receptor, serpin B3, serpin B4, squamous cell carcinoma antigens, thrombospondin, tumor antigen 4, tumor-associated glycoprotein 72, tyosinase, tyrosine kinases, etc.

Also disclosed herein is a method of inducing apoptosis of regulatory T (Treg) cells in a subject in need thereof comprising: administering to the subject an effective amount of a pharmacutical composition comprising planetary ball milled (PBM) nanoparticles containing a cytotoxic agent and a cell targeting agent. The cell targeting agent may be folate or a folate derivative.

Also disclosed herein is the use of folate-coated biodegradable PBM nanoparticles containing more than one chemotherapeutic agent to treat cancer progression. Docetaxel- or carboplatin-loaded folate-coated PBM nanoparticles enhance tumor therapy efficacy, reduce toxicities, and increase tumor immunity by targeting and inducing BrCa cell and Treg cell apoptosis.

Also disclosed herein is targeted delivery of curcumin-loaded PBM nanoparticles coated with folate to enhance *in vivo* efficacy and improve current chemoprevention strategies against cancers.

Also disclosed herein is treating a subject with the PBM nanoparticles containing a cytotoxic agent in conjunction with the treatment of the subject beforehand, at the same time, or afterward with a therapeutically effective amount of at least one other therapeutic agent, including, but not limited to, curcumin or a chemotherapeutic agent that is not contained in PBM nanoparticles. The chemotherapeutic agent that is not contained in PBM nanoparticles may be selected from carboplatin, cisplatin, docetaxel, and oxaliplatin.

The pharmacuetical composition of the present application may be administered to the subject with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. The pharmacuetical composition of the present application may be administered directly to a tumor or cancer tissue, including administration directly to the tumor bed during invasive procedures. The pharmacuetical composition of the present application may also be placed on a solid support such as a sponge or gauze for administration to the affected tissues. PBM nanoparticles containing a cytotoxic agent of the invention can be administered in the usually accepted pharmaceutically acceptable carriers. Acceptable carriers include, but are not limited to, saline, buffered saline, and glucose in saline.

The appropriate dosage ("therapeutically effective amount") of the pharmacuetical composition of the present application will depend, for example, on the disease to be treated, the severity and course of the condition, whether the pharmacuetical composition of the present application is administered for preventive or therapeutic purposes, previous therapy, the patient's clinical history and response to the cytotoxic agent contained in the pharmaceutical composition, the type of cytotoxic agent used, and the discretion of the attending physician. The pharmacuetical composition of the present application can be suitably administered to the patent at one time or over a series of treatments and may be administered to the patent at any time from diagnosis onwards. The pharmacuetical composition of the present application may be administered as the sole treatment or in conjunction with other drugs or therapies useful in treating the condition in question.

In some embodiments, the pharmacuetical composition of the present application contains curcumin and is administrated at a daily curcumin dosage range of 0.1 mg/kg to 100 mg/kg, 0.5 mg/kg to 100 mg/kg, 0.1 mg/kg to 50 mg/kg, 0.5 mg/kg to 50 mg/kg, 1.0 mg/kg to 20 mg/kg, 2.5 mg/kg to 50 mg/kg, 2.5 mg/kg to 20 mg/kg and 5 mg/kg to 10 mg/kg. The curcumin-containing PBM nanoparticles may be administered continuously, hourly, four times a day, three times a day, two times a day, daily, or every 2, 3, 4, 5, 6 and 7 days, or every 1, 2, 3 or 4 weeks.

As a general proposition, the therapeutically effective amount of each cytotoxic agent contained in the PBM nanoparticles, such as curcumine or cisplatin, is administered in the range of about 1 ng/kg body weight/day to about 100 mg/kg body weight/day whether by one or more administrations. In a particular embodiments, each cytotoxic agent contained in the PBM nanoparticles is administered in the range of from about 1 ng/kg body weight/day to about 10 mg/kg body weight/day, about 1 ng/kg body weight/day to about 1 mg/kg body weight/day, about 1 ng/kg body weight/day to about 100 µg/kg body weight/day, about 1 ng/kg body weight/day to about 10 µg/kg body weight/day, about 1 ng/kg body weight/day to about 1 µg/kg body weight/day, about 1 ng/kg body weight/day to about 100 ng/kg body weight/day, about 1 ng/kg body weight/day to about 10 ng/kg body weight/day, about 10 ng/kg body weight/day to about 100 mg/kg body weight/day, about 10 ng/kg body weight/day to about 10 mg/kg body weight/day, about 10 ng/kg body weight/day to about 1 mg/kg body weight/day, about 10 ng/kg body weight/day to about 100 µg/kg body weight/day, about 10 ng/kg body weight/day to about 10 µg/kg body weight/day, about 10 ng/kg body weight/day to about 1 µg/kg body weight/day, 10 ng/kg body weight/day to about 100 ng/kg body weight/day, about 100 ng/kg body weight/day to about 100 mg/kg body weight/day, about 100 ng/kg body weight/day to about 10 mg/kg body weight/day, about 100 ng/kg body weight/day to about 1 mg/kg body weight/day, about 100 ng/kg body weight/day to about 100 µg/kg body weight/day, about 100 ng/kg body weight/day to about 10 µg/kg body weight/day, about 100 ng/kg body weight/day to about 1 µg/kg body weight/day, about 1 µg/kg body weight/day to about 100 mg/kg body weight/day, about 1 µg /kg body weight/day to about 10 mg/kg body weight/day, about 1 µg /kg body weight/day to about 1 mg/kg body weight/day, about 1 µg /kg body weight/day to about 100 µg/kg body weight/day, about 1 µg /kg body weight/day to about 10 µg/kg body weight/day, about 10 µg/kg body weight/day to about 100 mg/kg body weight/day, about 10 µg /kg body weight/day to about 10 mg/kg body weight/day, about 10 µg /kg body weight/day to about 1 mg/kg body weight/day, about 10 µg /kg body weight/day to about 100 µg/kg body weight/day, about 100 µg/kg body weight/day to about 100 mg/kg body weight/day, about 100 µg /kg body weight/day to about 10 mg/kg body weight/day, about 100 µg /kg body weight/day to about 1 mg/kg body weight/day, about 1 mg/kg body weight/day to about 100 mg/kg body weight/day, about 1 mg/kg body weight/day to about 10 mg/kg body weight/day, about 10 mg/kg body weight/day to about 100 mg/kg body weight/day.

In another embodiment, each cytotoxic agent contained in the PBM nanoparticles is administered in the range of about 10 ng to about 100 ng per individual administration, about 10 ng to about 1 µg per individual administration, about 10 ng to about 10 µg per individual administration, about 10 ng to about 100 µg per individual administration, about 10 ng to about 1 mg per individual administration, about 10 ng to about 10 mg per individual administration, about 10 ng to about 100 mg per individual administration, about 10 ng to about 1000 mg per injection, about 10 ng to about 10,000 mg per individual administration, about 100 ng to about 1 µg per individual administration, about 100 ng to about 10 µg per individual administration, about 100 ng to about 100 µg per individual administration, about 100 ng to about 1 mg per individual administration, about 100 ng to about 10 mg per individual administration, about 100 ng to about 100 mg per individual administration, about 100 ng to about 1000 mg per injection, about 100 ng to about 10,000 mg per individual administration, about 1 µg to about 10 µg per individual administration, about 1 µg to about 100 µg per individual administration, about 1 µg to about 1 mg per individual administration, about 1 µg to about 10 mg per individual administration, about 1 µg to about 100 mg per individual administration, about 1 µg to about 1000 mg per injection, about 1 µg to about 10,000 mg per individual administration, about 10 µg to about 100 µg per individual administration, about 10 µg to about 1 mg per individual administration, about 10 µg to about 10 mg per individual administration, about 10 µg to about 100 mg per individual administration, about 10 µg to about 1000 mg per injection, about 10 µg to about 10,000 mg per individual administration, about 100 µg to about 1 mg per individual administration, about 100 µg to about 10 mg per individual administration, about 100 µg to about 100 mg per individual administration, about 100 µg to about 1000 mg per injection, about 100 µg to about 10,000 mg per individual administration, about 1 mg to about 10 mg per individual administration, about 1 mg to about 100 mg per individual administration, about 1 mg to about 1000 mg per injection, about 1 mg to about 10,000 mg per individual administration, about 10 mg to about 100 mg per individual administration, about 10 mg to about 1000 mg per injection, about 10 mg to about 10,000 mg per individual administration, about 100 mg to about 1000 mg per injection, about 100 mg to about 10,000 mg per individual administration and about 1000 mg to about 10,000 mg per individual administration. The chemotherapeutic agent contained in the PBM nanoparticles may be administered daily, or every 2, 3, 4, 5, 6 and 7 days, or every 1, 2, 3 or 4 weeks.

In other particular embodiments, the amount of each cytotoxic agent contained in PBM nanoparticles administered is, or is about, 0.0006 mg/day, 0.001 mg/day, 0.003 mg/day, 0.006 mg/day, 0.01 mg/day, 0.03 mg/day, 0.06 mg/day, 0.1 mg/day, 0.3 mg/day, 0.6 mg/day, 1 mg/day, 3 mg/day, 6 mg/day, 10 mg/day, 30 mg/day, 60 mg/day, 100 mg/day, 300 mg/day, 600 mg/day, 1000 mg/day, 2000 mg/day, 5000 mg/day or 10,000 mg/day. As expected, the dosage will be dependant on the condition, size, age and condition of the patient. As expected, the dosage will be dependant on the condition, size, age and condition of the patient.

The PBM nanoparticles containing a cytotoxic agent may be administered, as appropriate or indicated, a single dose as a bolus or by continuous infusion, or as multiple doses by bolus or by continuous infusion. Multiple doses may be administered, for example, multiple times per day, once daily, every 2, 3, 4, 5, 6 or 7 days, weekly, every 2, 3, 4, 5 or 6 weeks or monthly. However, other dosage regimens may be useful. The progress of this therapy is easily monitored by conventional techniques.

Therapeutically effective amount of PBM nanoparticles containing a cytotoxic agent may be administered to a subject in need thereof as a sole therapeutic agent. The therapeutically effective amount of PBM nanoparticles containing a cytotoxic agent may kill or promote apoptosis of the tumor or cancer cells. The therapeutically effective amount of PBM nanoparticles containing a cytotoxic agent may inhibit or prevent the establishment of a tumor or cancer. The therapeutically effective amount of PBM nanoparticles containing a cytotoxic agent may inhibit or prevent the migration or metastasis of tumor or cancer cells from an existing tumor or cancer. The therapeutically effective amount of PBM nanoparticles containing a cytotoxic agent may inhibit or prevent the invasion of tumor or cancer cells into non-cancerous tissues.

Therapeutically effective amount of PBM nanoparticles containing a cytotoxic agent may be administered to a subject in need thereof in conjunction with one or more additional therapeutically effective cytotoxic agents. Said one or more additional therapeutically effective cytotoxic agents may be may be administered before, concurrently with, and/or after the PBM nanoparticles containing a cytotoxic agent.

The therapeutically effective amount of PBM nanoparticles containing a cytotoxic agent may augment the effectiveness of the one or more additional therapeutically effective cytotoxic agents in killing tumor or cancer cells. The therapeutically effective amount PBM nanoparticles containing a cytotoxic agent may reduce the amount of the one or more additional therapeutically effective cytotoxic agents required for killing tumor or cancer cellsThe therapeutically effective amount of PBM nanoparticles containing a cytotoxic agent may inhibit or prevent the migration or metastasis of tumor or cancer cells from an established tumor or cancer, enhancing the local effectiveness of the one or more additional therapeutically effective cytotoxic agents in killing tumor or cancer cells. The therapeutically effective amount of PBM nanoparticles containing a cytotoxic agent may inhibit or prevent the invasion of tumor or cancer cells into non-cancerous tissues, enhancing the local effectiveness of the one or more additional therapeutically effective cytotoxic agents in killing tumor or cancer cells.

In another embodiment, the PBM nanoparticles contain an agent that is cytotoxic to cancer cells. In some embodiments, the agent that is cytotoxic to cancer cells is selected from the group consisting of diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, a tricothecene, and fragments thereof.

The present invention is further illustrated by the following examples which should not be construed as limiting.

### REFERENCE EXAMPLE 1: PREPARATION OF PLANET BALL MILLING NANOPARTICLES

First, 10 to 15% (w/v) of alginate, cellulose, collagen, starch, lactose, other biopolymers or mixture thereof (excipient/biopolymer) was dissolved in water and mixed using a homogenizer. Next, 10 to 20% (w/v) of protein (*e.g*., BSA or IgG) or macromolecule (*e.g.,* gadolinium, paclitaxel, and cisplatin *etc.*) (w/v) was added to the excipient/biopolymer solution at 4 °C. Next, 10% of PEG (w/v) was added to the biopolymer-cytotoxic agent solution and stirred for 30 minutes. After centrifugation, the solution was poured into about 3 mm³ tablets and dried. These tablets were then milled using planetary ball milling under controlled temperature (<37 °C). The resulting particles were either used alone (*i.e.,* uncoated) or coated with a 5%, 10% or 20% PCL solution (in methylene chloride) by continuous stirring at 1000 rpm. The PCL-coated bio-polymer-PEG particles were finally rinsed with water, dried and stored as a powder.

*PCL Activation and Peptide Conjugation:* PCL was activated to attach peptide or substrates (*e.g*., folate) to its surface for targeted delivery of particles. First, 2 g of PCL was dissolved in 6 ml of dry dioxane and heated in a 50 °C. water bath for 2 hours to solubilize the polymer and then cooled at room temperature (RT). A 2 ml solution of N,N'disuccinimidylcarbonate (153.7 mg/ml in dry acetone) and 2 ml of a pyridine solution (4.745 mg/ml in dry acetone) was mixed with the PCL suspension with continuous stirring for 6 hours at RT. The mixture was next filtered using a G2 glass fiber with 1 µm pore size to remove precipitates. The resulting supernatant was precipitated with 4 volume of diethyl ether. The precipitant was resuspended in acetone and precipitated again with diethyl ether. The activated or reactive PCL was then dried and stored at 4 °C.

The activated PCL was then conjugated to lysine(s) or any free amine group from a peptide. The desired peptide or amino acid (*e.g.,* lysine) was dissolved in 0.1M Na.sub.2HPO₄, 0.1 M boric acid, pH 8.5 at a concentration of 2 mg/ml. Next, the activated PCL, dissolved in acetonitrile, was mixed with stirring to the peptide or amino acid at a ratio of 8:1 (activated PCL:peptide or amino acid) overnight at 4 °C. The excess activated and unconjugated PCL was removed by filtration using a glass filter.

*Coating of particles*: Particles of known quantity and size were coated with various concentrations (*e.g.* 5%, 10% and 10%) of PCL or amine-conjugated PCL dissolved in methylene chloride with continuous stirring (1000 rpm). The resulting PCL-coated particles were separated from the PCL coating solution by draining the supernatant after centrifugation. The resulting PCL coated nanospheres were strained, air-dried and stored at 4 °C.

*Thermal analysis*: Differential scanning calorimetry (DSC) was carried out using a PerkinElmer Diamond DSC and thermogravimetric/differential thermal analysis. First, .about.1 mg of particles were heated from 30 °C to 250 °C. at a constant rate (10 °C. per minute), in atmospheric nitrogen. A thermal gravimetric profile of the particles was then performed.

*Quantification and characterization of protein loading: In vitro* release studies of PCL-coated bio-polymer-PEG particles after incorporation of their contents were carried out in phosphate buffered saline pH 7.4 at 37 °C. Approximately, 100 mg of particles were suspended and digested in a 100 ml of PBS or citrated tris buffer at 37 °C. to determine their rate of release. Particles were also filtered using 0.1, 0.22, or 0.45 µm Millipore filter(s). Protein-containing contents were dissolved in either PBS or citrated tris buffer saline and the rate of release was measured using the Lowry's protein assay. In the case of polymer-coated particles, samples were collected every 48 hours, while replacing the PBS to mimic the infinite sink conditions of the host.

Circular dichroism spectrum analysis was also used to assess the regularity of molecular assemblies comparing standard or encapsulated protein (*e.g*., BSA or IgG). The conformations of the intra-molecular structures (*e.g.,* alpha helical, beta sheets, *etc*.) of the released protein were determined by measuring their circular dichroism, in terms of Δε, and comparing them to unencapsulated protein as standards.

*Particle size, charge and morphology analysis:* Particles were analyzed for their particle size by laser diffraction using Malvern particle size and charge analyzer (Zetasizer). Particles were dispersed in dH₂O and analyzed for charge and size. The surface morphology of the PCL-coated bio-polymer-PEG particles was characterized by scanning electron microscopy (SEM). For SEM analysis, particles were prepared by dispensing the dried particles onto one side of a double adhesive tape, which was stuck to an aluminum stub. The stubs were then coated with gold using Polaron SC S00-sputter coater, to a thickness of 20 to 30 nm. The samples were then introduced into the specimen chamber of a scanning electron microscope and examined for surface morphology. The infrared spectra of the different stages of the PCL-coated bio-polymer-PEG particle formulations were obtained by first mixing 1 mg of the samples with 100 mg of dried potassium bromide powder. Next, infrared spectra of the samples were assayed using a Fourier transformed infra red spectrometer.

For sizing the nanoparticles, the following directions provide a guide. In general, particle size ranging from 5 to 30 nm, 30 to 180 nm, 0.2 to 1 µm, 1 to 6 µm, 4 to 12 µm or 10 to 60 µm can be controlled by using a 50 ml grinding jar filled with one to three 20 mm and ten to sixteen 10 mm balls along with the sample to be encapsulated and the excipient using grinding speed maintained at 200 to 400 rpm. This grind interval should be set for 10 minutes followed by a resting cycle for 15 minutes. Ten to twenty-five cycles (grind and rest) allow for >99.5% particles of size ranging from 5 to 30 nm, 30 to 180 nm, 0.2 to 1 µm, 1 to 6 µm, 4 to 12 µm or 10 to 60 µm. When the size of the grinding jar is increased to >50 ml (*e.g.,* 125 ml, 500 ml, *etc*.), the number of balls are increased proportional to the increase in jar volume.

*Examples of particle size modulation by grinding ball and active-resting cycles:* The particle size ranging from 5 to 30 nm can be obtained using a 50 ml grinding jar filled with three 20 mm and ten 10 mm balls along with the sample to be encapsulated and excipient, using grinding speed maintained at 400 rpm. This grind interval is set for 10 minutes followed by a resting cycle for 15 minutes. Fifteen to twenty-five cycles (grind and rest) result in >99.5% particles of size ranging from 5 nm to 30 nm.

Particle size ranging from 30 to 180 nm can be obtained using a 50 ml grinding jar filled with three 20 mm and ten 10 mm balls along with the sample to be encapsulated and the excipient, using the grinding speed maintained at 400 rpm. This grind interval should be set for 10 minutes followed by a resting cycle for 15 minutes. Fifteen to twenty cycles (grind and rest) result in >99.5% particles of size ranging from 30 to 180 nm.

Particle size ranging from 0.2 to 1 µm can be obtained by using 50 ml grinding jar filled with three 20 mm and ten 10 mm balls along with the sample to be encapsulated and excipient, using grinding speed maintained at 300 rpm. This grind interval should be set for 10 minutes followed by a resting cycle for 15 minutes. Fifteen to twenty cycles (grind and rest) result in >99.5% particles of size ranging from 0.2 to 1 µm.

Particle size ranging from 1 to 6 µm can be obtained by using 50 ml grinding jar filled with two 20 mm and thirteen 10 mm balls along with the sample to be encapsulated and excipient, using grinding speed maintained at 300 rpm. This grind interval should be set for 10 minutes followed by a resting cycle for 15 minutes. Twenty to twenty-five cycles (grind and rest) result in >99.5% particles of size ranging from 1 to 6 µm.

Particle size ranging from 4 to 12 µm can be obtained using 50 ml grinding jar filled with two 20 mm and thirteen 10 mm balls along with the sample to be encapsulated and excipient, using grinding speed maintained at 250 rpm. This grind interval should be set for 10 minutes followed by a resting cycle for 15 minutes. Fifteen to twenty cycles (grind and rest) result in >99.5% particles of size ranging from 4 to 12 µm.

Particle size ranging from 10 to 60 µm can be obtained by using 50 ml grinding jar filled with one 20 mm and sixteen 10 mm balls along with the sample to be encapsulated and excipient, using grinding speed maintained at 200 rpm. This grind interval should be set for 10 minutes followed by a resting cycle for 15 minutes. Ten to fifteen cycles (grind and rest) allow for >99.5% particles of size ranging from 10 to 60 µm.

*Effect of excipient*/*biopolymer concentration on particle characteristics:* Particles with diverse size (5 nm to 60 µm ± 10% of mean size) were produced using about 10% (w/v) of sodium alginate, about 8% (w/v) of cellulose or about 10% (w/v) of starch in dH₂O (excipient/biopolymer solutions). Below 2% (w/v) of sodium alginate, cellulose or starch, the yield of particles with a mean size that did not vary > 1 0% was found to be low. At greater than 15% of the sodium alginate or 10% of cellulose, the excipient/biopolymer solutions remained highly viscous and could not be used for tablet formation and subsequent particle preparation. Similarly, the starch (excipient/biopolymer) solution required heating to 50 °C for 20 minutes for optimal solubility and viscosity and the concentration of starch could not exceed 12% (w/v).

The viscosity of the excipient/biopolymer solutions also had a significant influence on the morphology of the particles. Particles became smoother and spherical with increasing concentrations of excipient/biopolymer solution (>1% (w/v)). However, <10% excipient/biopolymer solution produced the ideal size of particle. Optimum yield was achieved with the concentrations at 10%, 8%, or 10% (w/v) of sodium alginate, cellulose or starch, respectively, for the generation of nano- or micro-particles. Hence, in subsequent studies, these optimal concentrations were prepared for the generation of 3 mm³ tablets for milling into particles.

*Effect of milling speed and size of planetary balls on particle size:* The grinding speed of the planetary ball milling apparatus (Retsch PM100) and the size of the milling balls played a significant role in controlling the particle size, as indicated in the examples above. Varying the speed from 0 to 100 rpm for 20 minutes and using ten 10 mm plus three 20 mm balls, resulted in particles ranging 20 to 50 µm in size, while speeds 100 to 200 rpm for 20 minutes yielded 10 to 20 µm microparticles. Modulating speeds from 200 to 300 rpm for 20 minutes and using fifty 5 mm plus fifteen 10 mm balls resulted in particles 1 to 5 µm in size. Increasing speeds from 300 to 400 rpm and using one hundred 5 mm ball resulted in particles ranging 0.5 to 1 µm.

To generate particles <500 nm, tablets were first milled to produce microparticles -20 to 50 µm in size. The resulting microparticles were further milled at 400 to 600 rpm for 20 minutes using one hundred 3 mm plus five hundred 2 mm balls to produce nanoparticles ranging from 5 to 500 nm in size. Further increases in speed did not have any significant effect on size reduction.

*Particle protein entrapment and loading efficiencies*: BSA loading could be varied from 4 to 25% (w/v). The resulting particles formed after milling became aggregated and morphologically malformed at >20% (w/v) loading of protein. A maximum of about 20% BSA loading in the nano- or micro-particles could be achieved with 100% efficiency.

*Morphologies of uncoated and PCL-coated biopolymer-PEG particles:* is evident from SEM that the particles modified by PCL-coating appeared smooth and uniform compared to the uncoated particles. Additional smoothing of particle surface characteristics occurred with multiple PCL coatings. However, the surface of the particles changed from smooth and spherical to rough, non-spherical and vacuolated after *in vitro* content release.

### REFERENCE EXAMPLE 2: TUMOR ASSOCIATED ANTIGEN LIGAND-COATED AND CHEMOTHERAPEUTIC-LOADED PBM NANOPARTICLES ON BREAST CANCER CELLS

Cytotoxic drug-loaded biodegradable planetary ball milled (PBM) nanoparticles of ∼20 nm diameter are coated with polycaprolactone-polyethylene glycol copolymers conjugated to folate. The folate coated PBM nanoparticles are further loaded with docetaxel, carboplatin, or cisplatin. Incubation of primary cells and cell lines using 20 µM of cisplatin solution resulted in > 90% cell death of both BrCa cells (MCF-7, MDA-MB-231, and 4T1) and normal (primary) breast epithelial cells after 24 hr of culture. However, PBM nanoparticles containing 10X less chemotherapeutic agent were taken up and induced the same level of BrCa cell apoptosis as cisplatin, docetaxel, or carboplatin solution, respectively, but not primary breast epithelial cells. Also, breast tumor-bearing mice that received either cisplatin solution (8 mg/kg/week) or cisplatin-encapsulated folate-coated PBM nanoparticles (equivalent cisplatin dose = 0.08 mg/kg/week) by intravenous injection resulted in significant (and similar) tumor regression. Mice receiving the cisplatin solution had significantly higher kidney and liver toxicities than naive or PBM nanoparticle-treated mice. It is known that, similar to cancer cells that express high levels of folate receptor α (FRα/FR1), T regulatory (Treg) cells express FRγ (FR4) and FR4 blockades enhanced tumor immunity. Accordingly, the novel PBM nanoparticles of the present application enhance BrCa therapy efficacy, reduce toxicities, and increase breast tumor immunity.

PBM nanoparticle (containing carboplatin or docetaxel)-mediated tumor regression is characterized. Nanoparticles are used to treat MDA-MB-231-luciferase-positive (luc) as well as MCF-7-luc xenograft tumors (before and after development). A Caliper/Xenogen IVIS™ Biophotonic Imaging System may be used to quantify breast tumor burden. *Ex vivo* analysis of excised tumors by immunohistochemistry (IHC) and morphologic analysis determines changes in breast tumor apopotosis (caspase-3 and -9 positive) and proliferative (Ki67 positive) phenotypes, using Aperio Scanscope and Spectrum software.

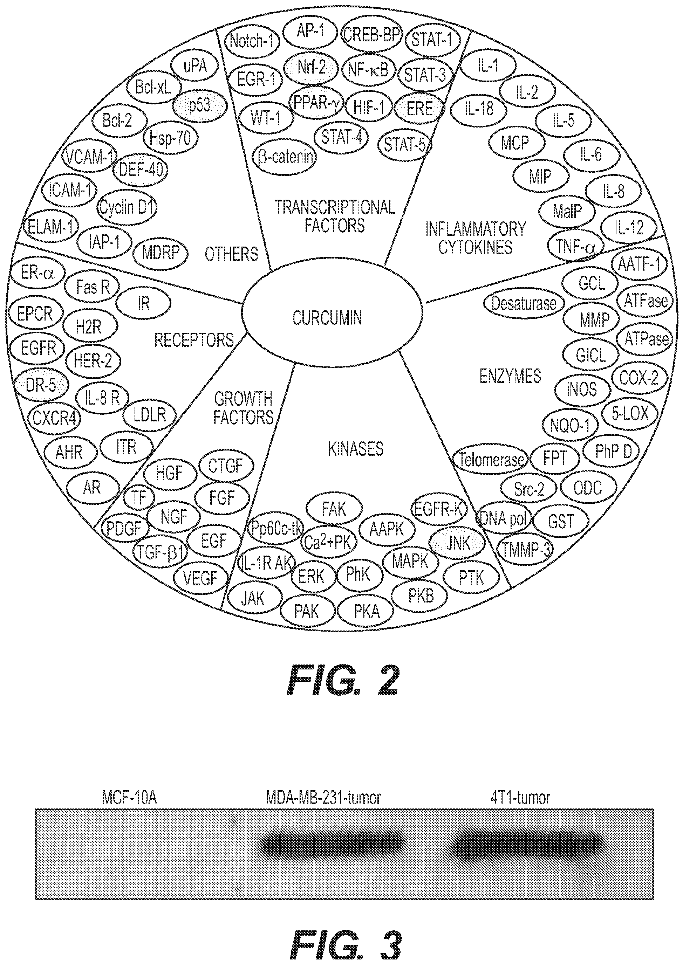

FIG. 3 shows that high affinity folate receptors (FRs) are expressed by BrCa tumors and cell lines.

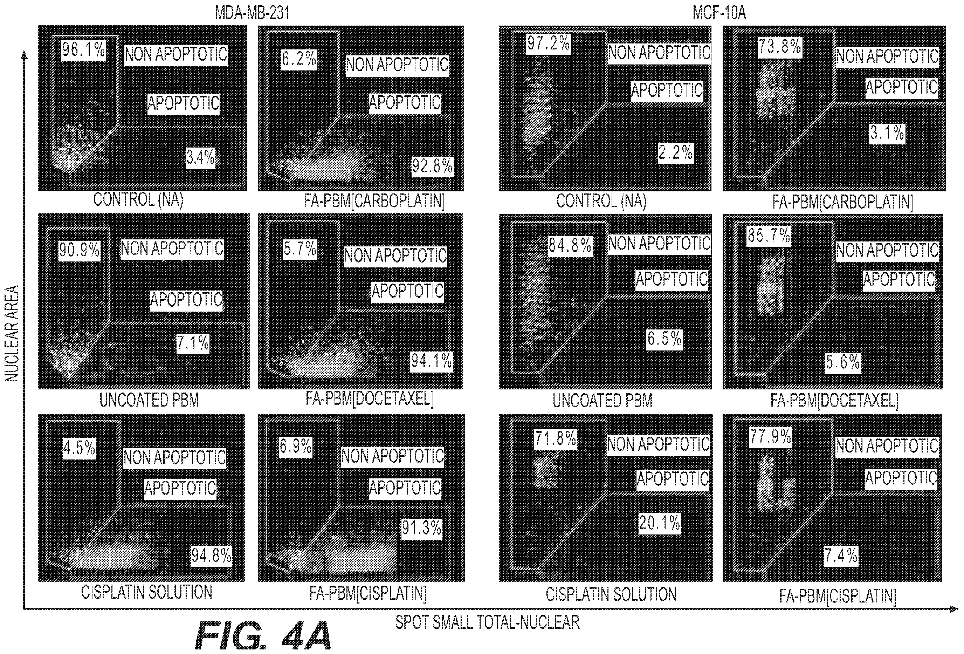

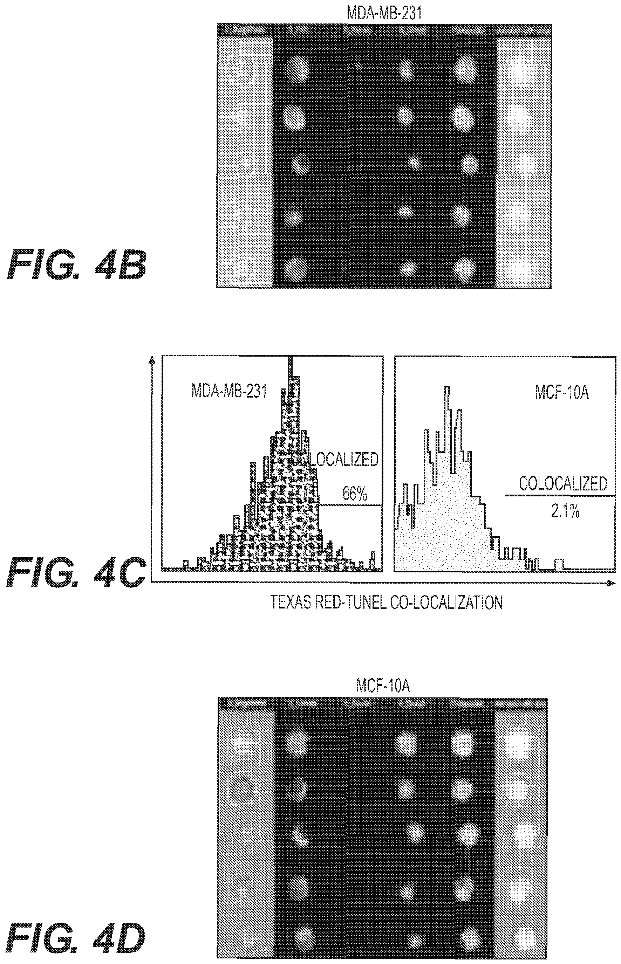

FIG. 4 shows that cisplatin-, docetaxel-, or carboplatin-loaded folate-coated PBM nanoparticles selectively target BrCa cells and induce apopotosis in them.

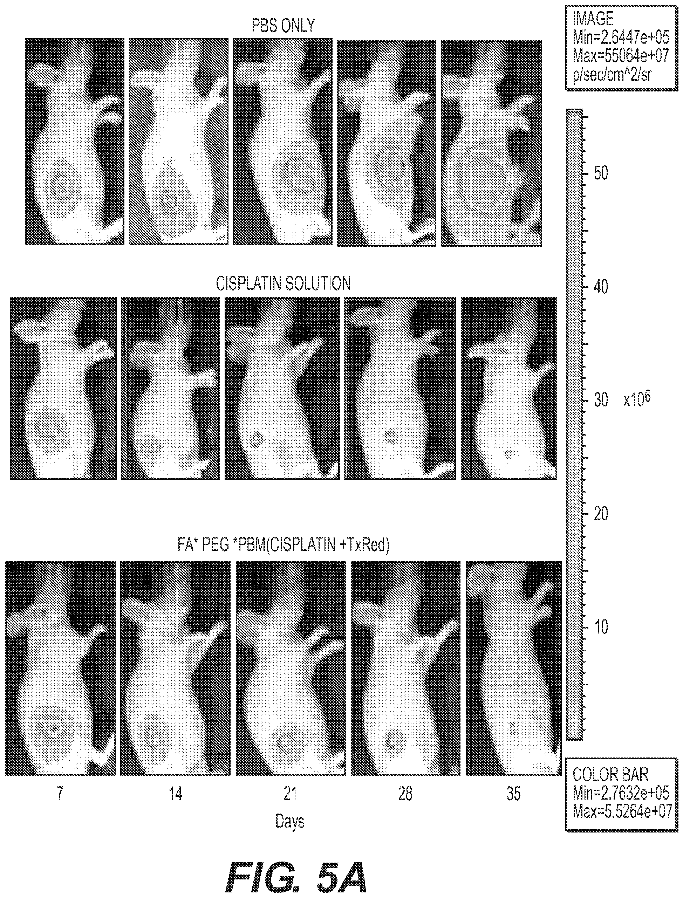

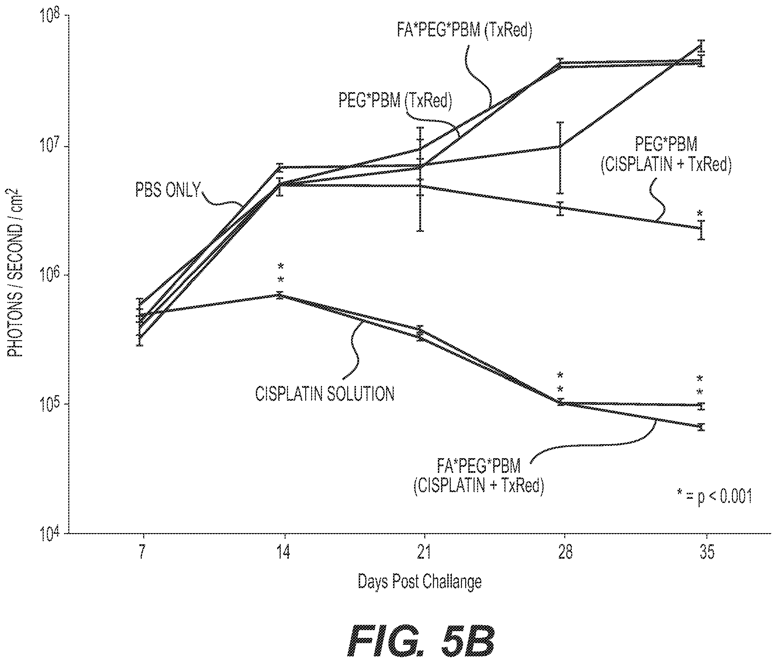

FIG. 5 shows that MDA-MD-231-luc tumor-bearing mice that received either cisplatin solution (8 mg/kg/week) or folate-coated PBM nanoparticles containing cisplatin (equivalent cisplatin dose = 0.08 mg/kg/week) by intravenous injection resulted in significant tumor regression. MDA-MD-231-luc tumor-bearing mice that received either cisplatin solution (8 mg/kg/week) or folate-coated PBM nanoparticles containing cisplatin (equivalent cisplatin dose = 0.08 mg/kg/week) by intravenous injection resulted in significant tumor regression.

Tumor-bearing mice receiving cisplatin solution had significantly higher kidney and liver toxicities than compared to naive or PBM nanoparticle-treated mice. PBM nanoparticle (containing carboplatin or docetaxel)-mediated tumor regression is characterized after orthotopic mammary fat pad injection of luciferase-expressing human BrCa cells (MDA-MB-231-luc; ER^{Neg} PR^{Neg} Her2^{Neg} and MCF-7-luc; ER^{Pos} PR^{Pos} Her2^{Pos}). Tumor progression or regression of tumors is followed by non-invasively imaging tumor-bearing Nu/Xid mice using the Caliper/Xenogen IVIS-100 imaging system after delivery of folate-coated PBM nanoparticles containing docotaxel, carboplatin, or no drug plus texas red dye. *Ex vivo* analysis by immunohistochemistry determines changes in breast tumor apopotosis (caspase-3 and -9) and phenotypes in the tumors treated with the novel PBM nanoparticles. NIH-III mice are used to graft MDA-MB-231-luc and MCF-7-luc cell lines.

PBM nanoparticle effects on tumor progression, immunity, and spontaneous tumor development are determined using 4T1-luc-synograft and novel PyMT-luc mouse models, respectively. Clinically relevant implanted (4T1) and spontaneous (PyMT) breast tumor development is non-invasively monitored using the Caliper/Xenogen IVIS-100 imaging system. Immunohistochemical and microscopic morphometric analysis using an Aperio histology scanner and analysis system quantify the changes in breast tumor apopotosis (caspase-3 and -9) and proliferative (Ki67) phenotypes. In addition, the number and activity of peripheral, lymph node and tumor-associated Treg cells are characterized for CD25, FR4, FoxP3, IL-10, and/or TGF-β1 expression and ability to suppress Th1/Th2 cell proliferation.

The PyMT-luc transgenic mice of the present application are established by first mating mice spanning 3 generations to create F3 MMTV-luc mice. F3 MMTV-luc mice are subsequently bred with PyMT mice to create PyMT-luc mice.

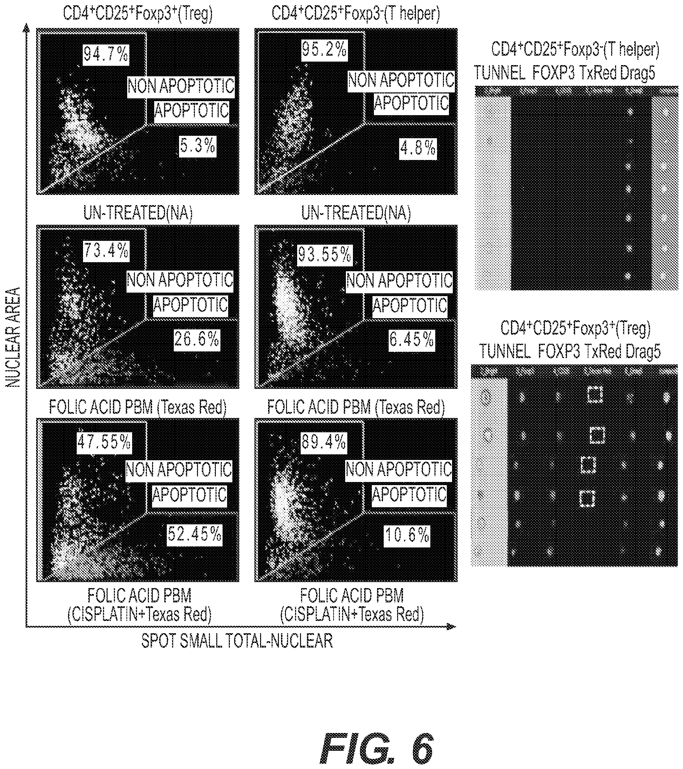

FIG. 6 shows that PBM nanoparticles selectively killed Treg (FoxP3⁺ CD25^{Hi}), but not T helper (FoxP3⁻ CD25^{Lo}) cells. Similar to cancer cells that express high levels of FR, T regulatory (Treg) cells express FRγ (FR4) and FR4 blockade enhanced tumor immunity; and (vi) show that we can selectively destroy Treg (FoxP3⁺ CD25^{Hi}) but not T helper cells. Together FIGS. 3-5 provide biological and clinical rationales to show that novel docetaxel- or carboplatin-loaded folate-coated PBM nanoparticles enhance tumor therapy efficacy, reduce toxicities, and increase tumor immunity by targeting and inducing BrCa and Treg cell death to reduce the health disparities associated with chemotherapy.

### REFERENCE EXAMPLE 3: EFFECT OF TUMOR CELL TARGETING AND CHEMOTHERAPEUTIC-LOADED PBM NANOPARTICLES ON PROSTATE CANCER CELLS

Cytotoxic drug-loaded biodegradable PBM nanoparticles of ∼20 nm diameter are coated with polycaprolactone-polyethylene glycol copolymers and folate. Treatment of both PCa cells (PC3 and PTEN-CaP2) and normal prostatic epithelial cells (PrEC and RWPE-1) using 20 µM of cisplatin resulted in > 90% cell death. PBM nanoparticles containing 100-fold less cisplatin were taken up and induced the same level of PCa cell apoptosis as cisplatin solution, but did not significantly affect normal cells. Tumor-bearing mice that received either cisplatin solution (8 mg/kg/week) or folate-coated PBM nanoparticles (containing an equivalent cisplatin dose of 0.08 mg/kg/week) resulted in significant (and similar) tumor regression. However, mice receiving the cisplatin solution had significantly higher kidney and liver toxicities than naive or PBM nanoparticle-treated mice. PCa cells express high levels of folate receptor α (FRα/FR1); similarly, T regulatory cells (Tregs) express FRγ/FR4 and FR4 blockade enhances tumor immunity. PBM nanoparticles enhance tumor therapy efficacy, reduce toxicities, and increase tumor immunity despite the health disparities associated with vitamin D deficiency, caveolin-1 expression, and NER genotypes.

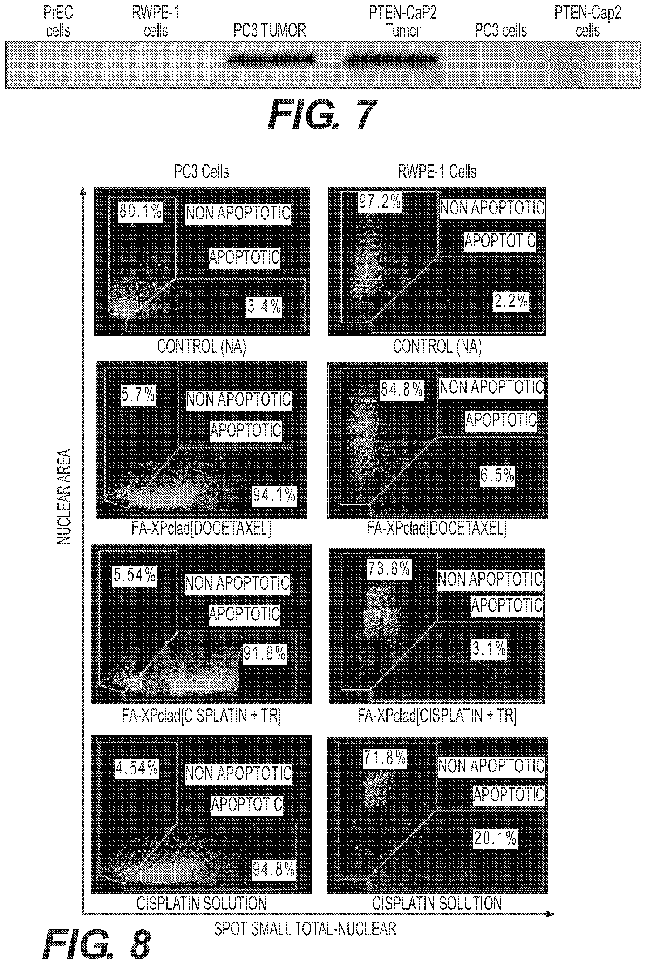

FIG. 7 shows that FRs are expressed by both PC3 and PTEN-CaP2 tumors, but not by the cell lines or by normal cells.

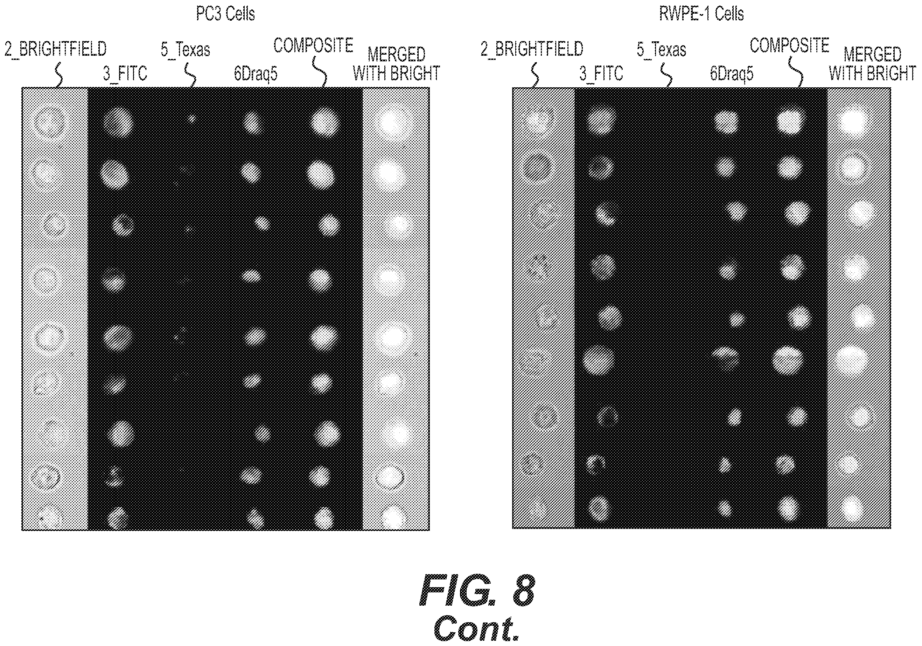

Cisplatin-loaded folate-coated PBM nanoparticles selectively targeted the destruction of PC3 cells *in vitro* as shown in FIG. 8.

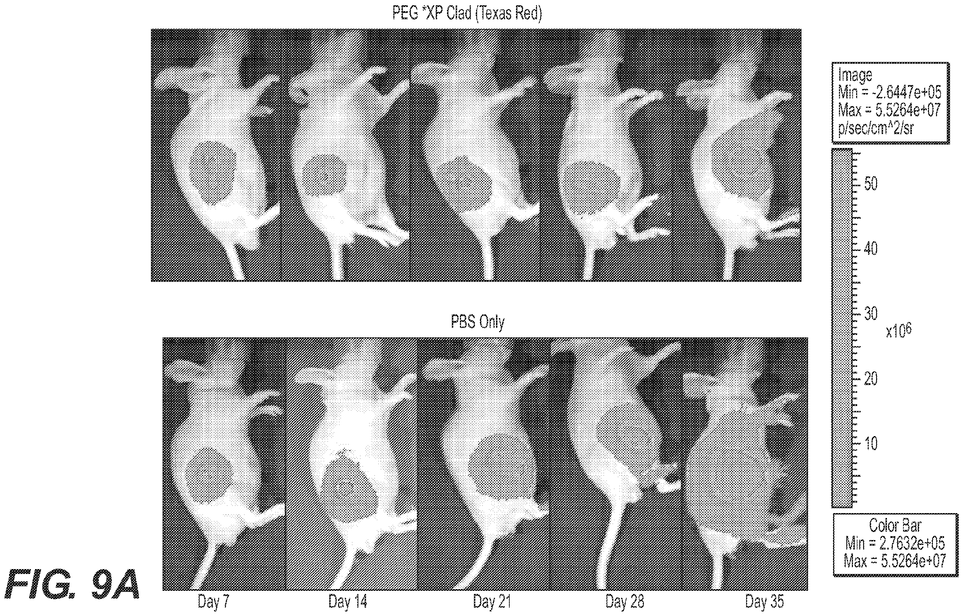

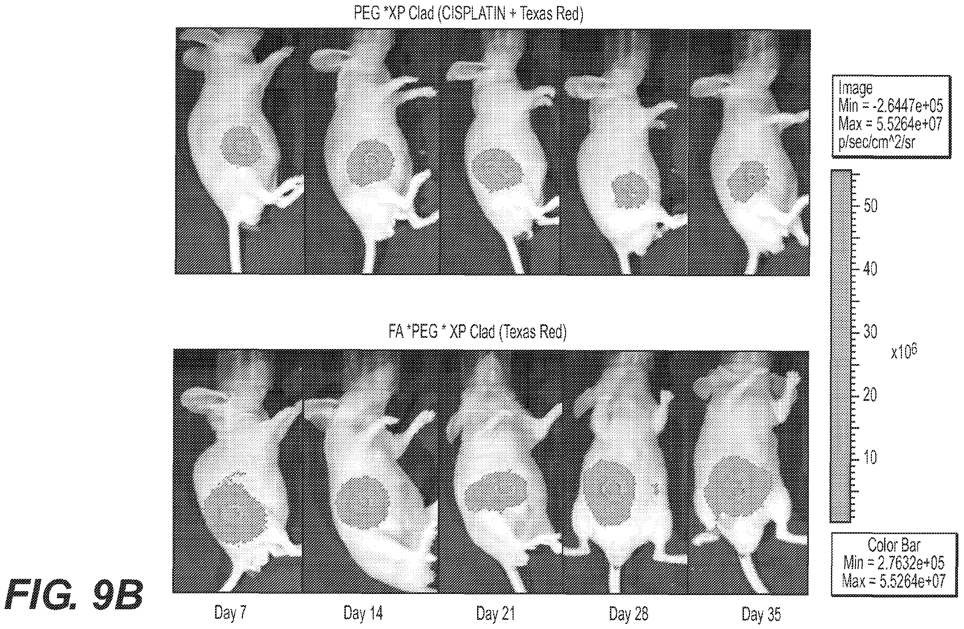

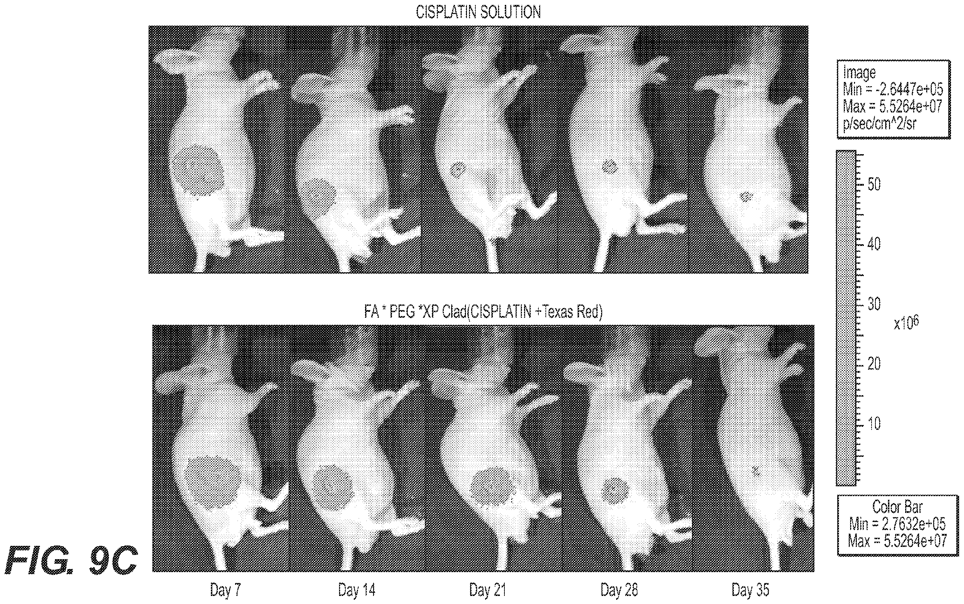

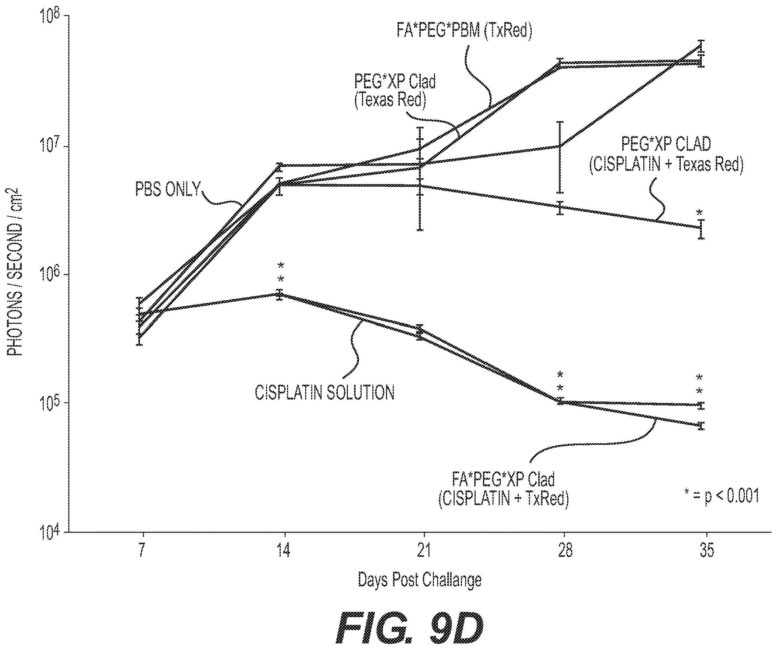

As shown in FIG. 9, PC3-luc tumor-bearing mice that received either cisplatin solution (8 mg/kg/week) or folate-coated XPclad© nanoparticles containing cisplatin (equivalent cisplatin dose = 0.08 mg/kg/week) by intravenous injection showed significant tumor regression. Importantly, however, mice receiving the cisplatin solution had significantly higher kidney and liver toxicities than compared to naive or XPclad nanoparticle-treated mice, as shown in Table 2.

**Table 2. Changes in blood chemistries after 6 doses (per week) of cisplatin solution or XPclad nanoparticles**

| **Serum Test** | **Normal Range** | **FA^{∗}PEG/PCL (cisplatin+TR)** | **Cisplatin solution** | **FA^{∗}PEG/PCL ('TR)** | **PEG/PCL (Cisplatin+TR)** | **PEG/PCl.(TR)** | **PBS** |
|---|---|---|---|---|---|---|---|
| Urea N₂ (mg/dl) | 20.9-40.5 | 38±6.93 | 36±0 | **44±3.46** | **44±3.46** | 40±6.93 | 40±3.46 |
| Creatinin (mg/dl) | 0.41-0.83 | 06±0 | 0.8±0.35 | 0.6±0 | 0.8±0.35 | 0.6±0 | 0.8±0.35 |
| Phosphorus (mg/dl) | 6.79-9.33 | 6.9±2.97 | 72±0 | 6.8±0.35 | 8±0.35 | **16.2±8.51** | **17.4±10.24** |
| Calcium (mg/dl) | 8.32-10.70 | 7.6±6.04 | **1.2±0** | 84.±0 | 8.2±0.35 | **1.2±0** | **1.2±0** |
| Na (mEq/l) | 154.5-167 | 150±12 | 152±3.46 | 158±346 | 156±0 | **138±12.00** | **132±6.00** |
| K (mEg/l) | 6.99-8.37 | 6.3±1.14 | **9.2±012** | 7.2±0 | **9.2±0.92** | **90.93±46.23** | **88.8±21.70** |
| Cl (mEq/l) | 116-127 | 100±9.17 | 106±3.46 | 104±3.46 | 102±0 | **90±6.00** | **88±3.46** |
| Bicarbonate (mmol/FL) | 20-30 | 21±8.65 | **10.2±1.04** | **12±3.6** | 20.53±0.81 | **10.2±1.20** | **9±0** |
| ALT(U/L) | 14.1-110.7 | 91.33±47.93 | **810±182.09** | 102±10.39 | 374±149.68 | **82±53.78** | 94±44.23 |
| ALT(U/L) | 49.6-171.2 | 396±123.98 | **808±199.75** | 316±19.29 | 984±216.25 | 1558±653.13 | 316±235.86 |
| Alk Phos (U/L) | 52.7-130.4 | 96±39.34 | **160±34.64** | 60±6 | 76±9.17 | 108±0 | 73.33±31.77 |
| CK(U/L) | 134-196 | 432±70.71 | **3975±45** | 2648±196.30 | **9114±1297.45** | **8660±142.51** | **2904±1011.3** |
| Cholestrol (mg/dl) | 87.8-147.3 | 88±24.98 | 84±.10.39 | 104±3.46 | 124±3.46 | 172±91.85 | 92±3.46 |
| Total Bilirubin (mg/dl) | 0.3±0.7 | 0.6±0 | 0.6±0 | 0.43±0.29 | 0.1±0.00 | 0.33±0.39 | 0.1±0 |
| Total protein (g/dl) | 4.99-6.27 | 5±0.92 | 5±0.35 | 21.2±28.41 | 5.4±0.00 | 5.2±0.35 | 4.8±0 |
| Albumin (g/dl) | 3.36-4.20 | 3.2±0.35 | 4.2±0.6 | 4±0.35 | 4.2±0.60 | 3.8±0.35 | 4±0.35 |
| Globulins (g/dl) | 1.5-2.5 | 1.8±0.60 | **1.2±0.6** | 1±0.35 | 1.6±035 | 1.8±0 | 1.4±0.35 |
| Albumin/Globulin ratio | 2-3 | 1.9±0.53 | 4.22±2.41 | 4.5±2.18 | 2.5±0.87 | 1.9±0.17 | 2.57±0.75 |
| Glucose (mg/dl) | 103-174 | 151±41.2 | **52±17.32** | **74±3.46** | **52±3.46** | **36±2** | **20±0** |
| Amylase (U/L) | 2280-2690 | 2272±317.62 | 2062±371.44 | **3206**±**175.58** | **3402±1553.68** | **3086±1518.32** | **3736±2740.16** |

| | | | | | | | |
|---|---|---|---|---|---|---|---|
| Significant change beyond normal ranges are highlighted in **BOLD.** | | | | | | | |

### EXAMPLE 4: EFFECT OF TUMOR ASSOCIATED ANTIGEN-TARGETING AND CURCUMIN-LOADED PBM NANOPARTICLES ON PROSTATE CANCER CELLS

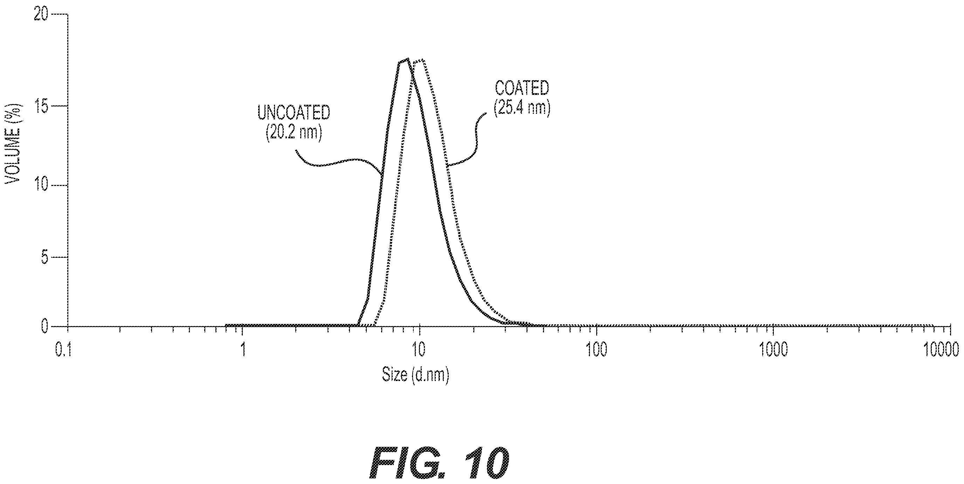

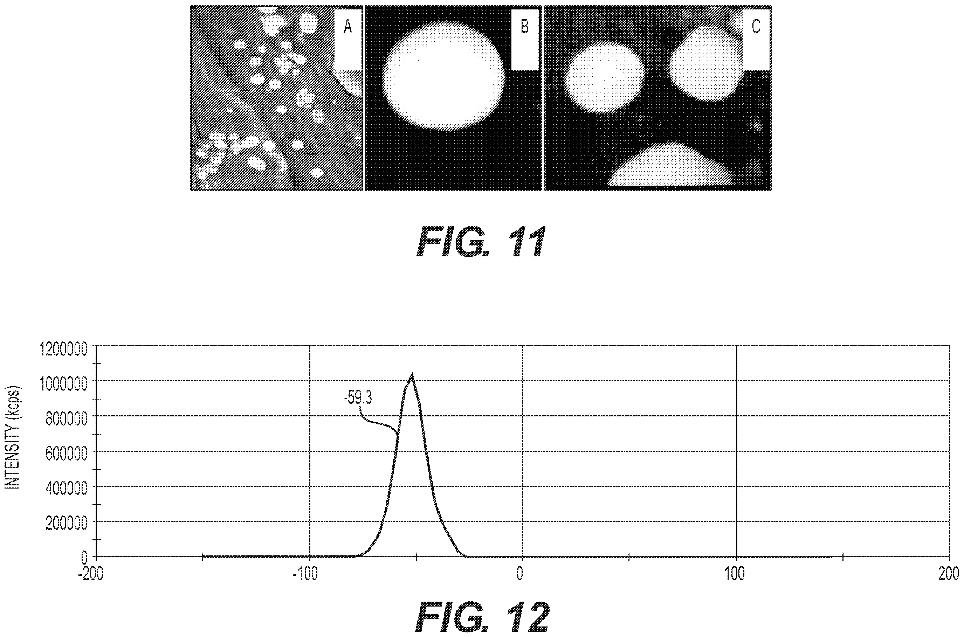

PBM nanoparticles of the present application have several advantages over existing nano-vehicles for the delivery of curcumin to cancer cells. The method of producing PBM nanoparticles is rapid (∼4 hours) and reproducibly (SD = 5 nm; LogP = -1.1 ± 0.3) creates ∼25 nm-sized nanoparticles, as shown in FIGS. 10 and 11. Curcumin-loaded biodegradable nanoparticles coated with polycaprolactone-polyethylene glycol copolymers conjugated to folate have ∼60 mV zeta potential as shown in FIG. 12.

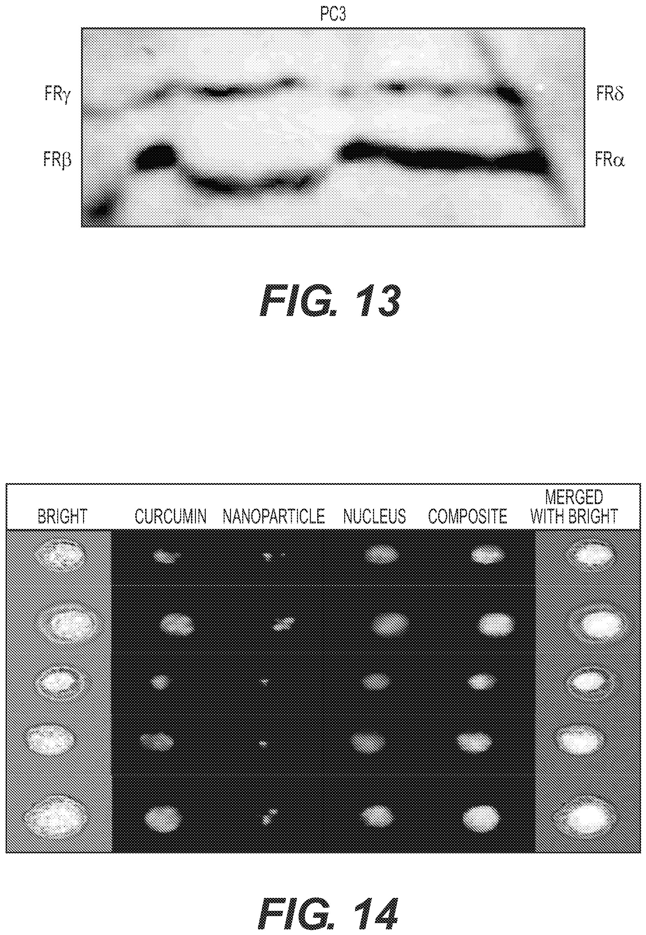

FIG. 13 shows the expression of FRs by PCa cells and PCa tumors established in SCiD mice.

Curcumin-loaded folate-coated PBM nanoparticles selectively target PCa cells and induce apopotosis, as shown in FIG. 14. Image Stream multispectral imaging by flow cytometry was used to measure the response of various PCa cells to treatments of both nano-curcumin and free curcumin. Our results indicate that the PBM formulation of curcumin was highly effective (70-85%) in killing cancer cells, even at concentrations lower than the IC₅₀ of free curcumin. Importantly, nanocarriers alone displayed practically no cytotoxicity. The preliminary data suggest PBM nanoparticles offer an innovative way to encapsulate curcumin to obtain more effective curcumin-mediated effects alone with specific tumor targeting via FRs.

### REFERENCE EXAMPLE 5: PBM NANOPARTICLE-MEDIATED TUMOR REGRESSION

PBM nanoparticle (containing oxaliplatin or docetaxel)-mediated tumor regression is characterized after intratibia injection of androgen-independent and luciferase-expressing human PC3 cells (PC3-luc; AR^{Neg} PTEN^{Neg}) and mouse PTEN-CaP2-luc (AR^{Pos} PTEN^{-/-}) given normal and vitamin D-deficient diets. To mimic advanced (bone metastasis) PCa, NIH-III or B6 male mice receiving vitamin D-deficient or normal diets are injected in left tibia with PC3-luc or PTEN-CaP2-luc cells, respectively. Tumor progression is followed non-invasively in non-castrated mice using a Caliper/Xenogen IVIS-100 imaging system. After tumors emit luminescence > 10⁵ photons/sec/cm², one set of mice is castrated to mimic androgen ablation and the second set is not. Subsequently, mice are intravenously treated every week with 0, 0.1 or 10 mg/kg of docetaxel or oxaliplatin, or folate-coated XPclad nanoparticles containing 0.1 mg/kg effective dose of docotaxel or oxaliplatin plus Texas red or no drug plus Texas red. Mice are imaged every week to monitor tumor progression/regression. When tumors in negative control (saline vehicle treated only) exceed 10⁸ photons/sec/cm², groups in each set are sacrificed. *Ex vivo* analysis by IHC is used to determine changes in prostate tumor apopotosis (caspase-3 and -9) or proliferative (Ki67) phenotypes in the tumors excised from mice treated with the novel XPclad nanoparticles. Microscopic and morphometric analysis determine the significance of treatments on prostate tumor apoptosis and change in the frequency or survival of PCa cells. Along with weekly bioluminescence imaging, blood chemistry and peripherial blood leukocyte subpopulations are monitored to evaluate tumor- or drug-associated toxicities. Blood samples are used to determine serum docetaxel and oxaliplatin PK/PD by mass spectrometry.

### Cell lines and cell culture

Two normal prostatic epithelial cell lines (PrEC and RWPE-1), one benign (PTEN-P2-luc), and two PTEN deficient and androgen-independent prostate carcinoma cell lines (PC3-luc and PTEN-CaP2-luc) are used in these studies. PrEC cells were obtained from Clonetics-Biowhittaker and cultured in PrEMB medium (Clonetics-Biowhittaker). RWPE-1 were obtain from ATCC and grown in keratinocyte serum free medium (K-SFM; Gibco) supplemented with factor 0.05 mg/ml of bovine pituitary extract and 5 ng/ml of epidermal growth factor. PC3-luc cells were obtained from Caliper/Xenogen. PC3-luc cells were cultured in Ham's F12K medium with 2mM L-glutamine and adjusted to contain 1.5 g/L sodium bicarbonate (ATCC) with 10% fetal bovine serum (FBS) (Sigma, St Louis, MO) at 37°C with 5% CO₂. After five passages in Ham's F12K media, PC3 cells were switched to RPMI-1640 with 10% fetal bovine serum FBS. PTEN-P2-luc and PTEN-CaP2-luc cells were kindly provided by Dr. Hong Wu. PTEN-P2-luc and PTEN-CaP2-luc cells will be cultured in DMEM supplemented with 10% fetal bovine serum, 25 ug/mL bovine pituitary extract, 5 ug/mL bovine insulin, and 6 ng/mL recombinant human epidermal growth factor (Sigma-Aldrich).

### Animals

NIH-III and B6 male mice are purchased from Jackson Laboratories. Animals arehoused and maintained in microbial isolator cages under conventional housing conditions at the Morehouse School of Medicine's animal facility. The guidelines proposed by the committee for the Care of Laboratory Animal Resources Commission of Life Sciences - National Research Council are followed to minimize animal pain and distress. Eight to ten week old mice are injected in the right tibia with 10⁶ PTEN-P2-luc (matched benign), PTEN-CaP2-luc (prostate adenocarcinoma), or PC3-luc cells. To assess the affects of hormone ablation on cytotoxic drug or PBM nanoparticle susceptibility, another group of mice is castrated after prostate tumor detection.

### PBM nanoparticle, docetaxel, and oxaliplatin treatments

After the establishment of primary tumor (measured by non-invasive imaging) with a net increase > 10⁵ photons/sec/cm², positive and negative control mice are intravenously injected every week with either PBS alone, 12 mg/kg or 0.12 mg/kg of docetaxel, and 12 mg/kg or 0.12 mg/kg of oxaliplatin in 100 µl of PBS. Mice weigh approximately 20g; hence, mice receive either ∼240 µg or ∼2.4 µg of cytotoxic drug. Experimental groups are intravenously administered every week with 60 µg of folate-conjugated or unconjugated PBM nanoparticles containing 0.4% w/w of sulforhodamine 101 acid chloride (Texas Red fluorescent dye; TxRed) alone, TxRed (0.4% w/w) + oxaliplatin (4% w/w), or TxRed (0.4% w/w) + docetaxel (4% w/w) in 100 µl of PBS (Prewett at el., Clinical Caner Research, 13:3432-3440 2007). Mice receive 100X less the recommended dose of docetaxel or oxaliplatin (*i.e.,* 2.4 µg instead of 240 µg per mouse) when delivered with the PBM nanoparticles.

### Analysis of PCa tumor progression, toxicities, and PK/PD

Every week for eight weeks, mice are injected with firefly luciferin 150 mg/kg (Caliper/Xenogen) by intraperitoneal injection using a 25 gauge x 5/8" needle. Tumor growth and metastasis of PC3-luc, PTEN-P2-luc and PTEN-CaP2-luc cell lines are assessed by non-invasively imaging mice using an IVIS Caliper/Xenogen imaging system. It is possible that individual cell lines have different copies of active luciferase (*luc*) gene. Subsequently, Pearson's moment correlation analyses is used to test the significance of the interrelationships between increase in photon expression, tumor growth and metastasis using Living Image software (Caliper/Xenogen). Serum is collected at sacrifice and urea N₂, creatine, P, Ca, Na, K, Cl, bicarbonate, alkaline phosphatase, alanine transaminase (ALT), aspartate aminotransferase (AST), creatine kinase (CK), cholesterol, total bilirubin, total protein, albumin, globulin, albumin/globulin ratio, glucose and amylase are measured. Docetaxel and oxaliplatin are quantified in serum samples collected weekly and sacrificed mouse organs by liquid chromatography mass spectrometry using the MSM Analytical Chemistry and Protein Profiling core facility.

### Immunohistochemistry, microscopy, and analysis

Paraffin-embedded excised tumor tissue from mice are sectioned (6 µm thick), mounted on positively charged superfrost slides (Fisher Scientific), and dried overnight. Sections are deparaffinized in xylene, followed by treatment with a series of alcohol [100%, 95%, and 80% ethanol/double distilled H₂O (v/v)] and rehydrated in PBS (pH 7.5). Sections are incubated with Tris-citrate target retrieval solution at 95°C for 20 min and allowed to cool for another 20 min, followed by sequential rinsing in Tris-buffered saline-Tween-20 (pH 7.6). Sections are incubated with 3% hydrogen peroxide for 12 min to block endogenous peroxidase and washed with PBS (pH 7.5). After washing with PBS, sections are incubated in protein blocking solution [5% normal human serum/0.5% normal goat serum in PBS (v/v)] for 20 min. The signal amplification system obtained from DAKO is used according to manufacturer's protocol. Briefly, slides are sequentially incubated with intervening washes for 15 min in the following: anti-AR (Millipore/Upstate), anti-caspase-3 (Cedarlane Laboratories), anti-caspase-9 (Santa Cruz), anti-CD4 (BioLegend), anti-chromogranin A (Zymed Laboratories), anti-cytokeratin 5 (Covance), anti-cytokeratin 8 (Covance), anti-FoxP3 (BioLegend), anti-IL-10 (BioLegend), anti-Ki67 (Novocastra Laboratories), and/or anti-synaptophysin (Zymed Laboratories) primary Abs diluted 1:200, biotinylated goat anti-rabbit Abs, streptavidin-biotin complex, amplification reagent, and peroxidase-labeled streptavidin (Vector Laboratories). Positive reactions are visualized by incubating the slides with diaminobenzidine/hydrogen peroxide (DAB; Sigma), as substrate(s). The sections are then rinsed with distilled water, counterstained with Gill's hematoxylin (Sigma), and mounted with Universal Mount (Research Genetics). Control samples are exposed to secondary Ab alone for non-specific staining.

All transverse sections of the whole prostate are scanned by a ScanScope GL system (Aperio Technologies) using a 40X objective followed by lossless compression and assessment of all immunostainings in identical anatomic regions. After images from the glass slides have been digitized, the resulting digital images are automatically analyzed using complex computer algorithms for standardized and unbiased controlled results. Morphometric analysis of sections are performed with the aid of Spectrum Plus software (Aperio Technologies). Automated image analysis is performed using Spectrum Plus algorithms for i) *Positive Pixel count and Color Deconvolution* for measuring and quantifying intensity per area for two or more stains (*e.g.,* increase in caspase-9 expression; ii) *Immunohistochemistry Membrane* image analysis for detection of membrane-associated staining for individual cells and quantifies intensity and completeness (*e.g.,* change in cytokeratin 5 and 8); iii) *Immunohistochemistry Nuclear* image analysis algorithm detects the nuclear staining for the individual nuclei and quantifies their intensity (*e.g.,* change in the number Ki67⁺ nuclei). Nuclei are classified as 0, 1+, 2+, and 3+ based on their intensity; iv) *Micromet* is specifically designed to detect micrometastasis of tumor cells in normal tissue.

### Tumor tissue and single cell isolation

Primary tumor and organs (*e.g.,* lung, liver, and bone) are surgically removed. Part of the tumor is fixed in formaldehyde for immunohistochemistry evaluation and the remainder is used to create single cell suspensions for subsequent Amnis ImageStream and flow cytometry analysis. Tumors in bone marrow are aspirated after cutting both ends using syringe. Secondary tumors from liver and lung are excised. Single cell suspensions of tumor cells are prepared mechanically using a scalpel and needle to tease apart the sample and release the cells into suspension. Fibrous tumors, which do not disaggregate well or yield poor cell suspensions, are subjected to digestion overnight with collagenase (type IV). Suspensions of recovered cells are washed in RPMI. Tumor cells are separated from other cell types by centrifugation on 55-75% discontinuous Ficoll-Hypaque density gradients (Pharmacia Fine Chemicals, Uppsala, Sweden). In brief, mixtures of cells (cells in 5 ml RPMI) are placed at the top of the gradient at 55% Ficoll-Hypaque, overlaid over 75% Ficoll-Hypaque, and centrifuged at 700 x g for 25 minutes at room temperature. Tumor cells from the top of the 55% Ficoll-Hypaque are collected and washed in RPMI, penicillin 50 IU/ml, streptomycin 50 pg/ml, gentamycin 50 pg/ml, HEPES buffer 10 mM, and L-glutamine 2 mM (complete medium).

### Interpretation of results

Optimal (10 mg/kg/week), but not suboptimal (0.1 mg/kg/week), therapeutic doses of docetaxel or oxaliplatin lead to PC3-luc and PTEN-CaP2-luc tumor regression, but also result in significant liver and kidney toxicities (*i.e*., elevated AST & ALT, hypocalcemia and hypoglycemia). Castration does not slow PC3-luc tumor growth, but initially slows PTEN-CaP2-luc tumor development that is followed by continued growth. Vitamin D deficiency has a deleterious effect on both docetaxel and oxaliplatin toxicity, but not the tumor regressing efficacy of these drugs. Both androgen-sensitive and hormone refractory PCa growth in mice given normal or vitamin D deficient diets are significantly inhibited by XPclad nanoparticles containing docetaxel or oxaliplatin. Oxaliplatin containing nanoparticles are more effective against PC3-luc than docetaxel, due to the higher susceptibility of AR^{Neg} cells to platinum-based therapies. In instances where suboptimal doses of cytotoxic drugs alone induce significant tumor regression the dose is lowered to 1.0 mg/kg and compared to a 10 µg/kg equivalent drug dose delivered by nanoparticles.

### REFERENCE EXAMPLE 6: PBM NANOPARTICLE EFFECTS ON SPONTANEOUS TUMOR AND Treg CELL DEVELOPMENT

PBM nanoparticle effects on spontaneous tumor and Treg cell development are examined using cPten^{-/-}PBcre4⁺luc⁺ or cPten^{-/-}PBcre4⁺luc⁺cav1⁺ (castrated or noncastrated) mice. P*ten*^{*floxed*/}*^{floxed}luc*⁺ without the *Cre* gene, which serve as negative tumor controls, and male cPten^{-/-}PBcre4⁺luc⁺ or cPten^{-/-}PBcre4⁺luc⁺cav1⁺ (castrated or noncastrated) mice are imaged every week during PCa development from murine PIN (6 weeks of age), invasive adenocarcinoma (> 9 weeks of age), and metastatic lymph node, lung and possibly bone at 12 weeks of age. After 9 weeks of age, tumors are allowed to progress to a net increase of 10⁵ photons/sec/cm² from luminescence detected in mice < 9 weeks of age. A set of mice is castrated to study androgen-independent hormone refractory PCa and another set is not castrated to further study the progression of PTEN-mediated PCa with androgens. Subsequently, mice are intravenously treated every week with 0 or 10 mg/kg of docetaxel or oxaliplatin, or folate-coated PBM nanoparticles containing 0.1 mg/kg effective dose of docotaxel or oxaliplatin plus texas red or no drug plus texas red. Mice are imaged every week to monitor tumor progression/regression. When tumors in negative control (saline vehicle treated only) exceed 10⁸ photons/sec/cm², groups in each set are sacrificed. *Ex vivo* analysis by immunohistochemistry is used to determine changes in prostate tumor apopotosis (caspase-3 and -9) and the prevalence of secretory luminal (CK8 and AR), basal (CK5), neuroendocrine (synaptophysin and chromogranin A)), and proliferative (Ki67) phenotypes in the tumors treated with the novel PBM nanoparticles. Microscopic and morphometric analysis determine the significance of treatments on prostate tumor apoptosis and change in the frequency or survival of secretory luminal, basal, neuroendocrine and proliferating PCa cells. In addition, the number and activity of peripheral, lymph node and tumor-associated lymphocytes are isolated and CD4⁺ T (CD3⁺) cells affinity purified and intracellularly and surface stained for flow cytometry analysis of Th1 (TNF-α, IFN-γ, LT), Th2 (IL-4 and IL-10), Th17 (IL-17A, IL-17-F, IL-22, and TNF-α), and Treg (IL-10, TGF-β, and/or FoxP3) cell frequency. The proliferation of purified T lymphocytes is measured after CD3 plus CD28 stimulation (*i.e.,* incubation on antibody-coated plates) with or without FR4 to assess Th1/Th2 cell proliferation.

### Isolation of CD4⁺ T lymphocytes

Mice are sacrificed by CO₂ inhalation. Primary tumor and lymph nodes are surgically removed. Secondary tumors from liver and lung are excised. Single cell suspensions of tumor cells are prepared mechanically using a scalpel and needle to tease apart the sample and release the cells into suspension. Fibrous tumors, which did not disaggregate well or those that yield poor cell suspensions are subjected to overnight digestion with collagenase (type IV). Suspensions of recovered cells are washed in RPMI. Tumor cells are separated from other cell types by centrifugation on 75-100% discontinuous Ficoll-Hypaque density gradients (Pharmacia Fine Chemicals, Uppsala, Sweden). In brief, mixtures of cells (cells in 5 ml RPMI) are placed at the top of the gradient at 75% Ficoll-Hypaque, overlaid over 100% Ficoll-Hypaque, and centrifuged at 700 x g for 25 minutes at room temperature. Lymphocytes are collected from the interface of 75% and 100% Ficoll-Hypaque and are washed in RPMI supplemented with 10% heat inactivated fetal calf serum, penicillin 50 IU/ml, streptomycin 50 pg/ml, gentamycin 50 pg/ml, HEPES buffer 10 mM, and L-glutamine 2 mM (complete medium). CD4⁺ T cells are isolated by negative selection using our Miltenyi Biotec AutoMACS system, according to the manufacturer's protocol.

### Intracellular cytokine and surface staining of lymphocytes

Fluorophore-conjugated rat anti-mouse CD4, CD25, FR4, IFN-γ, TNF-α, IL-17A-, IL-17F, IL-22, IL-4, TGF-β1, IL-10, and FoxP3 (BD-PharMingen) are used for immunofluorecence staining. Briefly, 10⁵ lymphocytes isolated from primary tumor and metastatic sites are washed in FACS buffer (2% bovine serum albumin in PBS). Cells are incubated with 0.5 µg per 10⁵ of Fc Block™ (BD-PharMingen) on ice for 30 minutes for blocking non-specific binding. Fc Block is removed by washing the cells with FACS buffer and cell pellets are incubated with fluorochrome conjugated rat-anti-mouse CD4, CD25 and/or FR4 Abs in 10 µl of primary Abs and 90 µl of FACS buffer for 40 minutes on ice. Excess Abs are removed by washing these cells three times in FACS buffer. For intracellular staining, these cells are re-suspended in saponin solution and washed in 1X Perm/Wash™ solution (Biolegend) for detection of intracellular (*e.g.,* cytokines) and nuclei (*e.g.,* FoxP3) markers. After permeabilization, cell pellets are incubated with 10 µl of primary Abs (rat anti-mouse [IFN-γ, TNF-α, and LT] or [IL-17A, IL-17F, and IL-22] or [IL-4, IL-5, and IL-10] or [TGF-β1, IL-10, and FOXP3]) and 90 µl of FACS buffer for 40 minutes on ice. Excess stains are removed by washing these cells three times in FACS buffer. Cells are resuspended in 70 µl of fixative (1% paraformaldehyle in PBS) and analyzed by flow cytometry.

### Ex vivo phenotype analysis by image analysis using the Amnis ImageStream System

Changes in the frequency CD4⁺ T cell populations or PCa tumor cells after FACS staining are analyzed using an Amnis ImageStream System, which allows for flow cytometry-based image acquisition and analysis with six channel (Bright filed, dark filed, and four channel for different fluorochrome). Images acquired are analyzed using Image Data Exploration and Analysis Software (IDEAS). This method confirms that Texas red positive nanoparticles are associated with luciferase positive PCa cell and FR4⁺ Treg cell apoptosis.

### Animals (spontaneous bioluminescent prostate adenocarcimona)

To generate mice with conditional inactivation of *Pten* alleles and activation of the luciferase reporter and caveolin-1 genes, *ARR2PB* promoter-driven prostate epithelium-specific *Cre* line PB-Cre4 (strain B6.D2-*Tg*^{*Pbsn-cre*/}*4Prb*; NCI-Frederick MMHCC), B6.129-Gt(ROSA)26Sortm2(ACTB-Luc)Tyj mice (LSL-Luc mice, Jackson Laboratory), and/or PBcav1⁺ mice are first bred to generate PBcre4⁺ luc⁺ and PBcre4⁺ luc⁺ cav1⁺ mice. Female homozygous offspring from these mice are then first bred with homozygous floxed *Pten* (stain C.129S4-*Pten^{tm1Hwu}*/J; Jackson Laboratory) mice. The male homozygous offspring carrying the PBcre4⁺ cPten^{-/-} luc⁺ and PBcre4⁺cPten^{-/-} luc⁺ cav1⁺ mice are used for the proposed treatment regimens. F2 generation of male PBcre4⁺ cPten^{-/-} luc⁺ and PBcre4⁺cPten^{-/-} luc⁺ cav1⁺ offsprings are used and above mentioned Power's analysis revealed that 10 *cPten*^{*-*/}*⁻L* mice in each group are needed to evaluate significance of treatment responses. Nonrecombinant littermates, *e.g. Pten*^{*floxed*/}*^{floxed}L* without the *Cre* gene, serve as controls. To assess the response of *Pten* null PCa to hormone ablation, another set of mice is castrated at 16 weeks (when invasive adenocarcinoma has formed) for the analysis of immediate (< 2 weeks) and extended (∼10 weeks) responses to androgen withdrawal.

### Analysis of PCa tumor progression

Every week for eight weeks, mice are injected with firefly luciferin 150 mg/kg (Xenogen) by intraperitoneal injection using a 25 x 5/8" gauge needle. Despite being homozygous for *Pten* gene deletion, the individual PBcre4⁺ cPten^{-/-} luc⁺ and PBcre4⁺cPten^{-/-} luc⁺ cav1⁺ mice will have different copies of active luciferase (*L*) gene. The copy number of trans L genes are determined and confirmed by multiplex polymerase chain reaction amplification and Southern blot analysis to insure the number of *L* genes responsible for luciferase will not change from experiment to experiment. The mice are imaged before the onset of PIN (*i.e*., < 6 weeks of age) and only the net increase > 10⁵ photons/sec/cm² will constitute the beginning of PCa in the PBcre4⁺ cPten^{-/-} luc⁺ and PBcre4⁺cPten^{-/-} luc⁺ cav1⁺ mice. Subsequently, Pearson's moment correlation analyses are used to test the significance of the interrelationships between increase in photon expression, tumor growth and metastasis using Living Image software (Caliper/Xenogen).

### Interpretation of Results

Optimal (10 mg/kg/week), but not suboptimal (0.1 mg/kg/week), therapeutic doses of cytotoxic drugs leads to tumor regression in PBcre4⁺ cPten^{-/-} luc⁺ mice, but not PBcre4⁺cPten^{-/-} luc⁺ cav1⁺ mice, along with significant liver and kidney toxicities (*i.e*., elevated AST & ALT, hypocalcemia and hypoglycemia). Castration initially slows tumor development, but will "relapse" and continue to grow albeit at a slower rate. Both androgen-sensitive and hormone refractory PCa progression are significantly inhibited by PBM nanoparticles containing docetaxel or oxaliplatin regardless of caveolin-1 expression levels in tumors. Docetaxel and oxaliplatin (alone or encapsulated) also result in fewer metastatic lesions in this model. While CK8^{Pos} AR^{Pos} luminal neoplasms and hyperplasia of the CK5^{Pos} basal cell compartment occur, *Pten* inactivation also leads to an expansion of neuroendocrine differentiation (*i.e*., synaptophysin^{Pos} chromogranin A^{Pos} and increasingly AR^{Neg}). Neuroendocrine cells are AR^{Neg} and thus are androgen-independent; hence, hormone refractory PCa could derive from a subpopulation of androgen-independent tumor cells that are present before hormone ablation therapy or cells that gain androgen independency due to subsequent genetic alterations and selection after castration. This potential differentiation is reduced by oxaliplatin or PBM nanoparticles containing oxaliplatin. As an alternative model to breeding of PBcre4⁺ cPten^{-/-} luc⁺ and PBcre4⁺cPten^{-/-} luc⁺ cav1⁺ mice, PTEN-CaP2-luc tumor cells made to overexpress caveolin-1 by expression vector are grafted into B6 mice.

### REFERENCE EXAMPLE 7: SYSTEMIC AND IMMUNE TOXICITIES IN PTEN-CAP2-LUC TUMOR PROGRESSION

Systemic and immune toxicities in PTEN-CaP2-luc tumor progression in castrated and non-castrated wild-type and Xpc^{-/-} mice due to docetaxel, oxaliplatin, and PBM nanoparticles are elucidated. To mimic advanced (bone metastasis) PCa in ERCC1 variant individuals, B6 and B6.Xpc^{-/-} mice are injected in left tibia PTEN-CaP2-luc cells. Tumor progression is followed non-invasively in non-castrated mice using our Caliper/Xenogen IVIS-100 imaging system. After tumors emit luminescence > 10⁵ photons/sec/cm², one set of mice is castrated (*i.e*., to mimic androgen ablation) and the second set is not. Subsequently, mice are intravenously treated every week with 0 or 10 mg/kg of docetaxel or oxaliplatin, or folate-coated PBM nanoparticles containing 0.1 mg/kg effective dose of docotaxel or oxaliplatin plus texas red or no drug plus texas red. Mice are imaged every week to monitor tumor progression/regression. When tumors in negative control (saline vehicle treated only) exceed 10⁸ photons/sec/cm², groups in each set are sacrificed. *Ex vivo* analysis by immunohistochemistry is used to determine changes in prostate tumor apopotosis (caspase-3 and -9) and the prevalence of secretory luminal (CK8 and AR), basal (CK5), neuroendocrine (synaptophysin and chromogranin A)), and proliferative (Ki67) phenotypes in the tumors treated with the novel PBM nanoparticles. The number and activity of peripheral, lymph node and tumor-associated lymphocytes are isolated and CD4⁺ T cells affinity purified and intracellularly and surface stained for flow cytometry analysis of Th1, Th2, Th17, and cell frequency as before. Specifically, the proliferation of purified T lymphocytes after CD3 plus CD28 stimulation with or without FR4 to access Th1/Th2 cell proliferation as before. Along with weekly bioluminescence imaging, blood chemistry and peripherial blood leukocyte subpopulations are monitored to evaluate tumor- or drug-associated toxicities that may be associated with Xpc deficiency.

### Interpretation of results

Optimal (10 mg/kg/week), but not suboptimal (0.1 mg/kg/week), therapeutic doses of docetaxel or oxaliplatin lead to PTEN-CaP2-luc tumor regression in both B6 and B6.Xpc^{-/-} mice. This reduction in tumor growth also coincides with significantly higher toxicities (*i.e*., elevated AST & ALT, hypocalcemia and hypoglycemia) in B6.Xpc^{-/-} mice than compared to B6 mice. Castration slows and possibly halts PTEN-CaP2-luc tumor development, but is followed by continued growth, in both B6 and B6.Xpc^{-/-} mice. Xpc deficiency does not have a deleterious effect on PBM nanoparticles (containing docetaxel or oxaliplatin)-mediated androgen-sensitive or hormone refractory PCa growth reduction. Importantly, PBM nanoparticles mediate tumor reduction in the absence of toxicity in both B6 and B6.Xpc^{-/-} mice. These findings coincide with higher tumor bioavailability or localization of docetaxel and oxaliplatin delivered by PBM nanoparticles, than compared to groups receiving cancer drug solutions. In an alternative model to PTEN-CaP2-luc tumor grafts, NIH-III Xpc^{-/-} are established mice to carryout these experiments using the PC3-luc xenograft model.

### EXAMPLE 8: CURCUMIN-LOADED PBM NANOPARTICLE-MEDIATED TUMOR REGRESSION

### PBM nanoparticle formulation, Synthesis of NHS-Activated Folate, DSC-Activated PEG, and generation of Folate-conjugated PEG

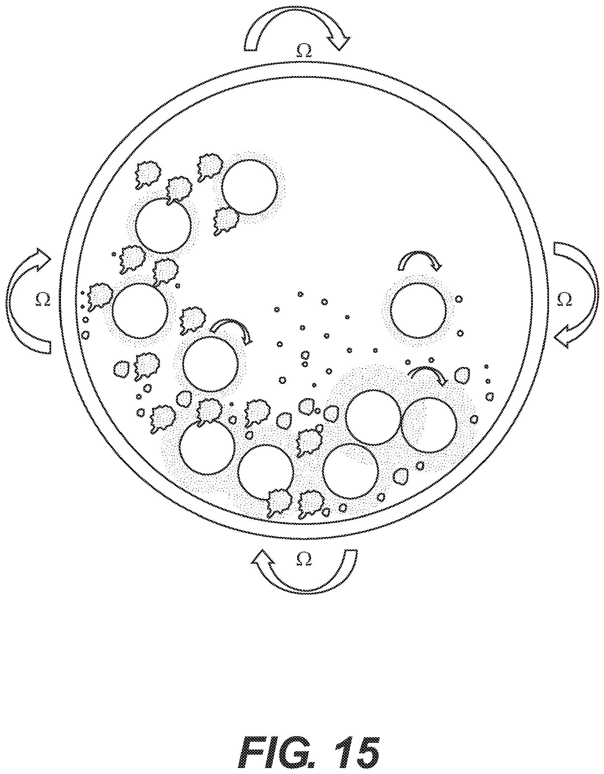

Curcumin loaded PBM nanoparticles are formed in a milling jar that holds heat absorbant zirconium oxide milling balls as shown in FIG. 15. The milling jar rotates about its own axis as well as in the opposite direction around a common axis of the chamber wheel. This produces the rotation of planetary balls and milling of the particles from the marcoparticles containing starch, polyetylene glycol, Texas red and curcumin.

### Minimum effective dose (MED), optimal dosing schedule (ODS) and safety for PBM nanoparticle

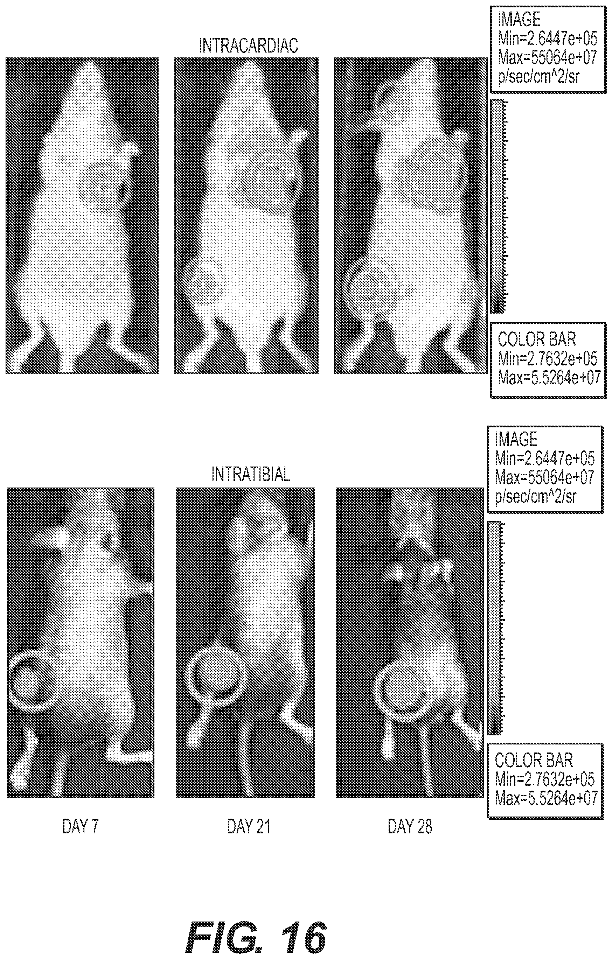

The MED determination and optimal schedule is evaluated in male athymic NIH-III mice injected intratibial with PC3-luc cells (FIG. 16). Dosing was initiated after the establishment of primary tumor (measured by non-invasive imaging) with a net increase >10⁵ photons/sec/cm². For the MED, mice were treated with dose levels of 10 µg, 20 µg, 50 µg, 100 µg, 200 µg and 400 µg of curcumin-loaded PBM nanoparticle per mouse, no treatment, and only nanoparticle (without curcumin) given by tail vein injection three times weekly for 28 days. For the optimum dosing schedule, three additional schedules were evaluated and compared to the control of three times weekly; daily (five days per week), every four days, and weekly for 28 days. All mice were weighed and tumor measurements taken three times weekly to evaluate toxicity, efficacy and safety of PBM nanoparticle.

### In vivo pharmacokinetic studies

Luciferase-expressing human androgen-refractory (PC3-luc; AR^{Neg}) cell line areinjected intratibial into six groups (n=10) of male nude mice weighing 25-30g. Direct injection of tumor cells into the bone marrow has been developed as an alternative to spontaneous metastases for the study of interactions between PCa and bone cells to mimic advanced bone metastasis. Group 1 is administered 20mg/mouse curcumin suspension; Group 2 is administered 200µg or best MED of curcumin-loaded nanoparticles with folate-conjugated. Group 3 is administered 200µg of curcumin-loaded nanoparticles with folate-unconjugated. Group 4 is administered 200µg of curcumin-loaded nanoparticles with Texas Red (to track intracellular location of particle ex vivo) + folate-conjugated. Group 5 is administered 200µg of curcumin-loaded nanoparticles with Texas red + folate-unconjugated. Group 6 is administrated with PBS only as negative control. Blood samples (0.5ml) are collected from the retro-orbital plexus under mild ether anesthesia into heparinized microcentrifuge tubes. After each sampling, 1 ml of dextrose normal saline is administered to prevent changes in the central compartment volume and electrolytes. Plasma is separated by centrifuging blood samples at 4000 rcf for 10 min at 4 °C. 25 µl of 2.8% of acetic acid is added to 25 µl of plasma, (for stability of curcumin) and 50 µl of Internal Standard (IS) 17-β estradiol acetate will be added and vortexed for 20s. After centrifugation at 10,000 rcf for 10 min, the organic layer is separated, which contained curcumin and IS. The residue left is reconstituted in 125 µl of methanol and analyzed by HPLC.

### Analysis of PCa tumor progression and toxicities

Every week, mice are injected with 150 mg/kg of luciferin (Caliper) by intraperitoneal injection using a 25 gauge x 5/8" needle. Tumor growth and metastasis are assessed by non-invasively imaging mice using an IVIS Caliper imaging system. Pearson's moment correlation analyses is used to test the significance of the increase in photon expression, tumor growth and metastasis using Living Image software (Caliper). Serum is collected at sacrifice and urea N2, creatine, P, Ca, Na, K, Cl, bicarbonate, alkaline phosphatase, alanine transaminase (ALT), aspartate aminotransferase (AST), creatine kinase (CK), cholesterol, total bilirubin, total protein, albumin, globulin, albumin/globulin ratio, glucose and amylase are measured using a blood chemistry analyzer (Boehringer Mannheim / Hitachi 911).

### Particle size, topography and zeta potential verification

Particle size is measured by dynamic light scattering using a Nano ZS (Malvern) taking the average of five measurements and zeta potential is estimated on the basis of electrophoretic mobility under an electric field as an average of 30 measurements. The surface morphology of nanoparticles is analyzed by atomic force microscopy (AFM) (Veeco Bioscope II), attached with Nikon eclipse TE 2000-S microscope, using Nanoscope software. Nanoparticle suspension is placed on silicon wafers with the help of a pipette tip and allowed to dry in air. The microscope is vibration damped and measurements are made using commercial pyramidal Si3N4 tips (Veeco's). Cantilever is used for scanning with a nominal force constant of 0.1 N/m. Images are obtained by displaying the amplitude signal of the cantilever in the trace direction, and the height signal in the retrace direction with both signals is simultaneously recorded.

### In vitro release kinetics of PBM curcumin nanoparticles

Curcumin-encapsulated PBM nanoparticles (100mg) are dispersed in 10 ml of phosphate buffer saline (PBS), pH 7.4, and divided in 20 microfuge tubes. Tubes are kept in a thermostable water bath set at room temperature. Free curcumin is completely insoluble in water; therefore, at predetermined intervals of time, the solution is centrifuged at 3000 rpm for 10 minutes to separate released (pelleted) curcumin from nanoparticles. The released curcumin is dissolved in 1ml of ethanol and 450 nm absorbance measured by spectrophotometry. The concentration of the released curcumin is calculated using a standard curve of curcumin in ethanol and the equation: Release (%)= [Curcumin] rel / [Curcumin] tot × 100 where, [Curcumin] rel is the concentration of released curcumin collected at time t and [Curcumin] tot is the total amount of curcumin entrapped in the nanoparticles.

Analysis of serum and liver enzyme levels provides information if any of the doses chosen is toxic and such doses are eliminated for further studies. The highest tolerated dose is used in the following example for efficacy study.

### EXAMPLE9: IN VIVO EFFICACY OF CURCUMIN-LOADED PBM NANOPARTICLES DIRECTED AGAINST PCa AND MECHANISMS OF CELL DEATH

Tumor implantation is through intracardiac (Bone metastasis, exhibit mainly osteoblastic characteristics), injection of luciferase expressing human androgen independent (PC3-luc; AR^{Neg} PTEN^{Neg}) PCa cell lines into the left ventricle of male athymic NIH-III mice as shown in FIG. 16. After the establishment of primary tumor (measured by non-invasive imaging) with a net increase > 10⁵ photons/sec/cm², negative and positive control mice are intravenously injected every week with either PBS alone, or 20mg/mouse curcumin suspension alone. *Ex vivo* analysis by immunohistochemistry is used to determine changes in prostate tumor apoptosis (caspase-3 and -9) and proliferative (Ki67) phenotypes in the tumors treated with novel PBM nanoparticles.

### Cell lines and cell culture

PC3-luc cells were obtained from Caliper/Xenogen. PC3-luc cells were cultured in Ham's F12K medium with 2mM L-glutamine and adjusted to contain 1.5 g/L sodium bicarbonate (ATCC) with 10% fetal bovine serum (FBS) (Sigma, St Louis, MO) at 37°C with 5% CO₂.

### Animals and treatments

NIH-III male mice are purchased from Jackson Laboratories. Animals are housed and maintained in microbial isolator cages under conventional housing conditions at the Morehouse School of Medicine's animal facility. After the establishment of primary tumor (measured by non-invasive imaging) with a net increase > 10⁵ photons/sec/cm², negative and positive control mice are intravenously injected every week with either PBS alone, or 20 mg/mouse curcumin alone. Experimental groups are intravenously administered every week with 200 µg of curcumin (w/w)-loaded nanoparticles with folate-conjugated or unconjugated, nanoparticle containing 0.4% w/w of sulforhodamine 101 acid chloride (Texas Red fluorescent dye; TxRed) alone, TxRed (0.4% w/w) + curcumin (4% w/w) with folate conjugated, or TxRed (0.4% w/w) + curcumin (4% w/w) without folate conjugated in 100 µl of PBS. Hence, mice receive 100 fold less curcumin (*i.e*., 200 µg instead of 20 mg per mouse) when delivered with the PBM nanoparticles. Subsequently, Pearson's moment correlation analyses is used to test the significance of the interrelationships between photon expression, tumor growth and metastasis using Living Image software (Caliper Life Sciences).

### Immunohistochemistry, microscopy, and analysis

Paraffin-embedded excised tumor tissue, kidney, pancreas, and liver from mice are sectioned (6 µm thick). Slides are processed using standard protocol and stained for anti-caspase-3 (Cedarlane Laboratories), -caspase-9 (Santa Cruz), -FR2 and -FR4 (Santa Cruz), and/or -Ki67 (Novocastra Laboratories). All transverse sections are scanned by a ScanScope GL system (Aperio Technologies) using a 40X objective followed by lossless compression and assessment of all immunostainings in identical anatomic regions. Morphometric analysis of sections is performed with the aid of Spectrum Plus software (Aperio Technologies). Automated image analysis is performed using Spectrum Plus algorithms for i) Positive Pixel count and Color Deconvolution; ii) Immunohistochemistry Membrane; iii) Immunohistochemistry Nuclear; and iv) Micromet.

The efficacy of the PBM nanoparticle and free curcumin is determined, based on their ability to inhibit the tumor growth. Mice receiving folate-coated PBM nanoparticle containing curcumin leads to tumor regression in mice without liver or kidney toxicities and fewer metastatic lesions in intracardiac challenge models. Free curcumin (unencapsulated) has low intrinsic activity, poor absorption, and/or rapid elimination and clearance from the body that will create the poor bioavailability. Folate coated targeted delivery of PBM nanoparticles increases curcumin efficacy as well as bioavailability.

### EXAMPLE 10: CHARACTERIZATION OF THE MOLECULAR MECHANISMS OF CELL DEATH BY CURCUMIN NANOPARTICLE

Curcumin decreases the proliferative potential and increases apoptotic potential of prostate cancer cells *in vitro,* largely by modulating the apoptosis suppressor proteins and by interfering with the growth factor receptor signaling pathways as exemplified by the EGF receptor. It also significantly alters microfilament organization and cell motility in PC-3 and LNCaP human prostate cancer cells *in vitro.* This study demonstrates that curcumin nanoparticle exerts significant effects on the actin cytoskeleton in prostate cancer cells, including altering microfilament organization and function. This may represent an important mechanism by which curcumin functions as a chemopreventative agent, and as an inhibitor of angiogenesis and metastasis.

Mechanisms of action of PBM nanoparticle on prostate cancer cell lines, and compared the functional pathways impacted by PBM nanoparticle to what has been previously reported for free curcumin. A principal cellular target of curcumin in cancer cells is activated nuclear factor kappa B (NF-κB), with many of the pleiotropic effects of curcumin being ascribed to inhibition of this seminal transcription factor. Electrophoretic mobility shift ("gel shift") assays are used to assess for the DNA binding ability of NF-κB. The ability of PBM nanoparticles to inhibit pro-inflammatory cytokines in peripheral blood mononuclear cells (PBMCs) is also determined. Many of these cytokines (IL-6, IL-8, and TNFα) have been implicated in the carcinogenesis process, including the induction of angiogenesis. Incubation of stimulated PBMCs with both free and PBM nanoparticle and measure mRNA levels of IL-6, IL-8 and TNFα, compared to DMSO and void nanoparticle-treated cells with evidence to test dose dependent reduction of IL-6 by both agents. Further, *in vitro* methods are used to determine whether curcumin-loaded nanoparticles induce caspase-dependent or - independent apoptotic signal(s) mediate prostate cancer cell death. Briefly, androgen-responsive (LNCaP) and androgen-refractory PCa cells (PC3) are grown in the presence of curcumin alone (10µM) as well as folate-coated and non coated curcumin loaded (10nM)-nanoparticle for 24, 48 and 72 hours with or without TNF and Fas inhibition. Apoptosis and/or necrosis is measured by TUNEL and flow cytometry. RT-PCR and Western blot analyses are also performed to determine the expression of the pro-apoptotic and pro-survival molecules, *e.g*., CD95, FasL, TNF, TNF-R1, NF-κB, Bax, Bcl-2, Bcl XL, cytochrome C, and caspases-9 and -3.

### Cell proliferation assay

A cell proliferation assay is performed using different concentrations of curcumin nanoparticle and free curcumin. MTT (3-[4,5- dimethylthiazol-2-yl]-2,5-diphenyl tetrazolium bromide) cell proliferation kit (Boehringer Mannheim) is used determined the cell proliferation using manufacturer's protocol.

### Apoptosis assays

An apoptosis assay is performed by Annexin V surface staining of treated cells using Vybrant® Apoptosis Assay Kit 3 (Alexa Fluor 488 annexin V/propidium iodide (Invitrogen), according to manufacturer's instructions.

### Western blot analysis

Briefly, cells are washed twice in PBS and lysed in RIPA lysis buffer. Total protein is determined by the Bradford assay. Equal amount of protein is loaded and resolved on 15% SDS-polyacrylamide gel and the proteins are transferred to a nitrocellulose membrane. The membrane is blocked with 5% non-fat dry milk and probed with respective primary and secondary antibodies. Final detection is performed using BCIP/NBT substrate (Promega, USA). The bands are analyzed and quantified using Image J software (NIH) and normalized with β actin control. The density of control is taken as 1 and results of treatment will be expressed in relation to the control as relative unit (RU). Actin polymerization and pseudopod formation in PCa cell lines are performed after treatment with free curcumin or PBM nanoparticle to test the cell motility and its role in cancer metastasis using Image Stream analysis.

Encapsulation of curcumin in PBM nanoparticles increases the bioavailability of curcumin and the 10nM curcumin loaded PBM nanoparticle lead to hormone refractory (PC3) PCa cell line apoptosis, blockade of NF-κB and downregulation of proinflammatory cytokines (IL-6, IL-8, and TNFα) *in vitro* compared to 1µM of free curcumin. Further, curcumin-containing nanoparticles are more effective against LNCaP because curcumin sensitizes androgen-responsive TRAIL-resistant cancer cells and targeted delivery of PBM nanoparticles increases the bioavailability of curcumin, further enhancing the efficacy of curcumin and higher cell motility (actin polymerization and pseudopod formation).

### REFERENCE EXAMPLE 11: TARGETING PROSTATE CANCER CELLS WITH PEPTIDE-CONJUGATED, DRUG-CONTAINING NANOPARTICLES

Cisplatin (Cispt) and TexasRed (TR)-encapsulated and cancer cell-binding peptides (I, II, III, and IV)- or folate-conjugated polycaprolactone-coated nanoparticles were prepared using method described above. The amino acid sequence of the peptides are as follows:
Peptide I - HTIRYDWHFTAR (SEQ ID NO:1)
Peptide II - FRPNRAQDYNTN (SEQ ID NO:2, see also EP1531159 and Zitzmann et al. Clinical Cancer Research, 2005, 11:139-146).
Peptide III - TIHYDFWHTRRA (SEQ ID NO:3)
Peptide IV - NFTRPNNYRDAQ (SEQ ID NO:4)

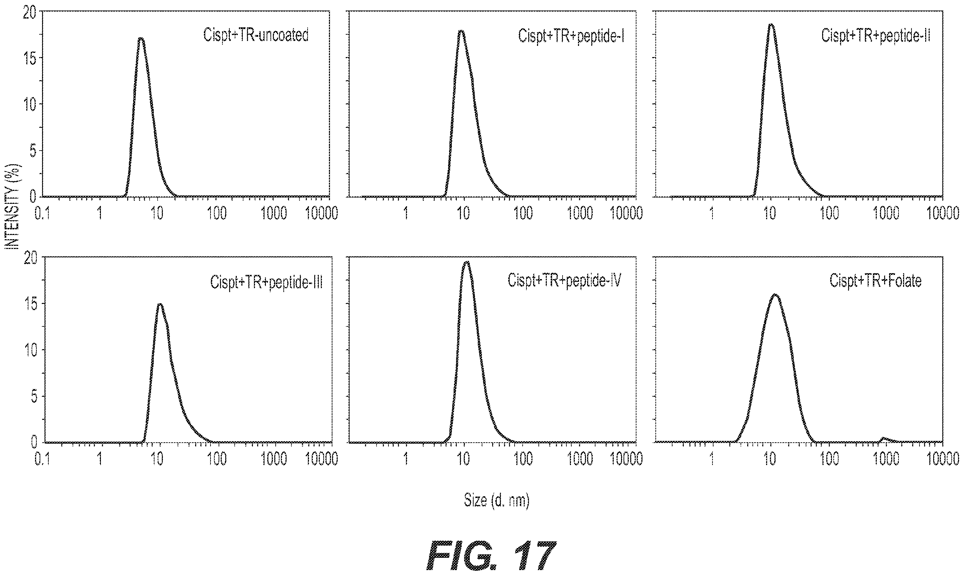

*Size distribution of prostate tumor-specific PBM nanoparticles.* The size distribution of the particles was measured using a Malvern ZetaSizer NS instrument using 0.1 mg/L concentration (5% mass, assuming a density of 1g/cm3) of nanoparticles. FIG. 17 showsarticle size distribution of prostate tumor-specific PBM nanoparticles.

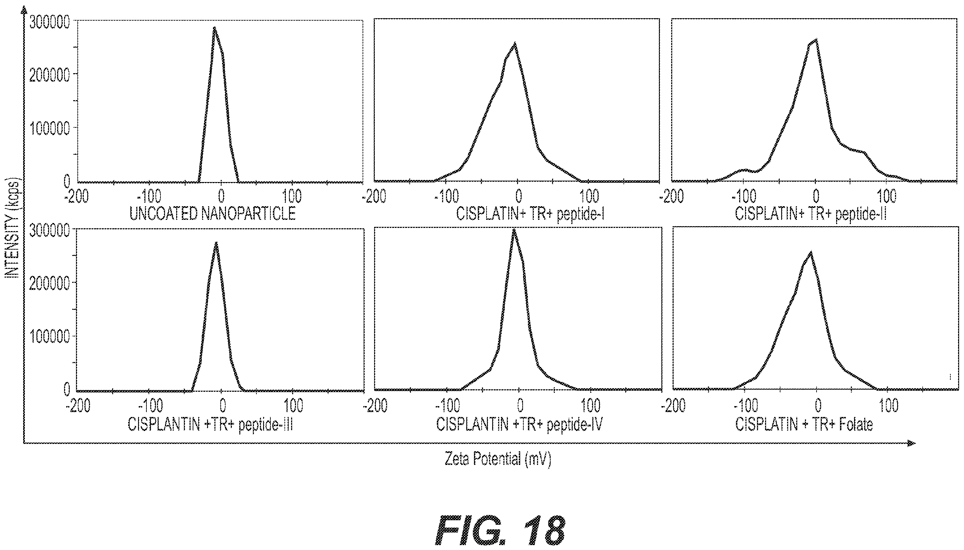

*Zeta potential of prostate tumor-specific PBM nanoparticles.* Cisplatin (Cispt)- and TexasRed (TR)-encapsulated and cancer cell-binding peptides (I, II, III, and IV)- or folate-conjugated polycaprolactone-coated nanoparticle zeta potential distribution was measure using a Malvern ZetaSizer NS, using 0.1 mg/L concentration (5% mass, assuming a density of 1g/cm3) of nanoparticles. FIG. 18 shows the Zeta potential of prostate tumor-specific PBM nanoparticles.

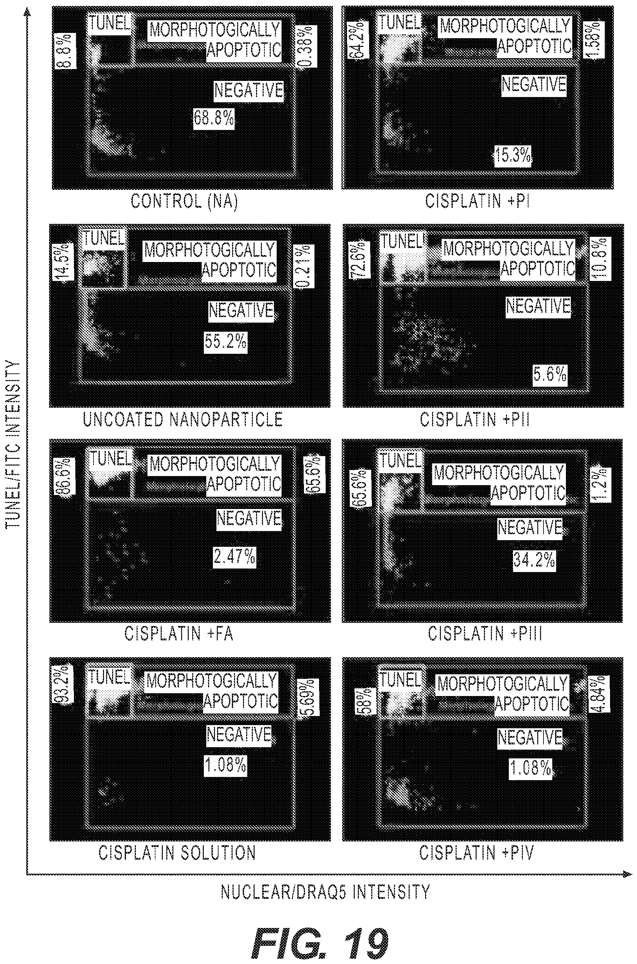

*Tunel and DRAQ5 staining of PBM nanoparticle-treated cancer cells.* PC3 cells were treated with 2 µM of cisplatin solution alone, cisplatin-containing and cancer cell-binding peptides (I, II, III, and IV)- or folate-conjugated polycaprolactone-coated PBM nanoparticles, or uncoated nanoparticles. PBM particles were allowed to adhere for 1 hour and non-adherent particles were removed by washing 5 times with PBS. As a positive control, cells were also treated with cisplatin solution (20 µM). Next, cells were incubated for 48 hours at 37°C with 5% CO2. Subsequently, cells were fixed and stained for Amnis ImageStream analysis with the Avidine-FITC and DRAQ5. FIG. 19 shows Tunel and DRAQ5 staining of PBM nanoparticle-treated cancer cells. Identification of apoptosis by nuclear morphometry. Tunel (FITC) intensity is plotted on the Y axis and nuclear DRAQ5 intensity on the X axis. Non-Apoptotic events are shaded red, true apoptotic events in light green and morphologically normal cells associated with apoptotic nuclear fragments in dark green.

*PBM nanoparticle-mediated cancer and normal cell apoptosis.* DU145 (prostate cancer cell line) and PrEC (primary prostatic epithelial cells) cells were treated with cisplatin solution or cisplatin-containing and cancer cell-binding peptides (I, II, III, and IV)- or folate-conjugated polycaprolactone-coated PBM nanoparticles, or uncoated nanoparticles. Cells are treated with cisplatin solution concentration(20uM) and nanoparticle with concentration(2uM)along with different peptides and folate, after treatments incubate the cells for 1 hour and than wash five times with PBS and again incubate cells for 48 hours in 370C with 5% CO2, fixed and stained the cells with the Avidine-FITC and Fluorescent DNA binding dye DRAQ5 and imaged on the Image stream system.

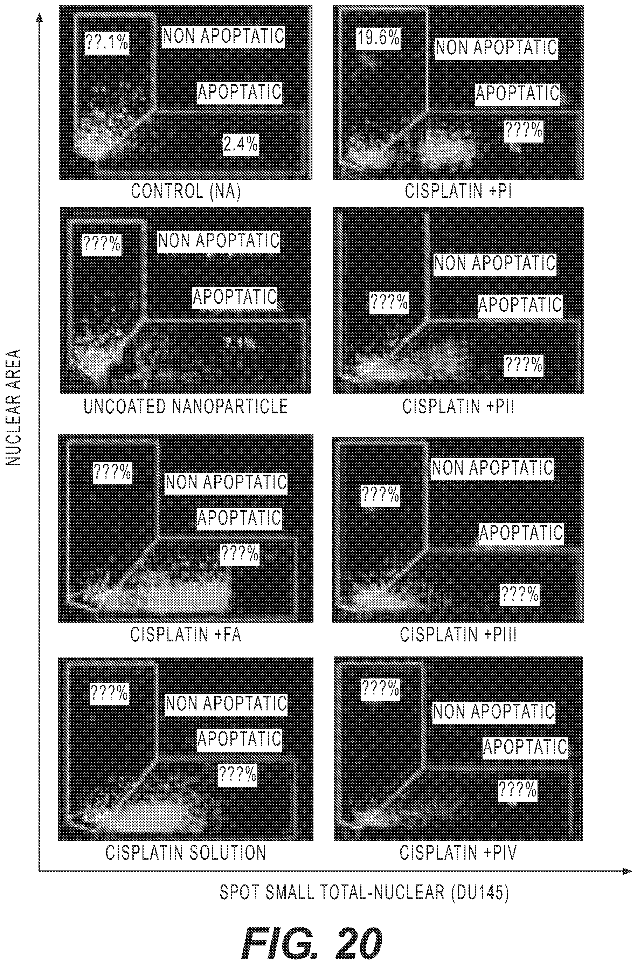

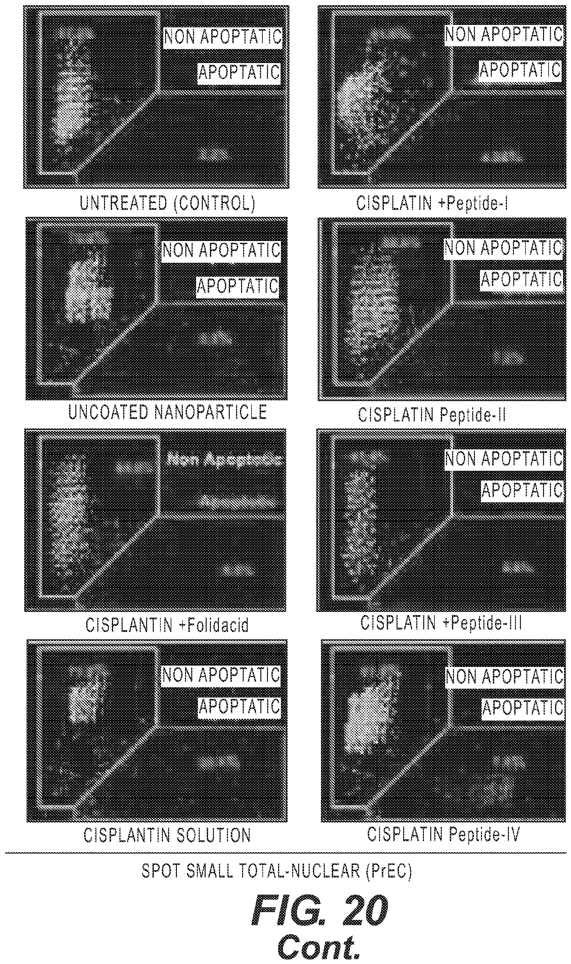

FIG. 20 shows PBM nanoparticle-mediated cancer and normal cell apoptosis. Apoptotic cells were identified using two standard image based features from the IDEAS package: Area and Spot Small Total. Condensed apoptotic nuclei have lower nuclear area value as compared to live cells. Also images of fragmented apoptotic nuclei exhibit small, bright regions with higher spot small total values as compared to the uniform images of normal nuclei. While cisplatin solution lead to the apoptosis of both DU145 and PrEC cells, targeted PBM nanoparticle cisplatin formulations only lead to apoptosis of DU145 cells.

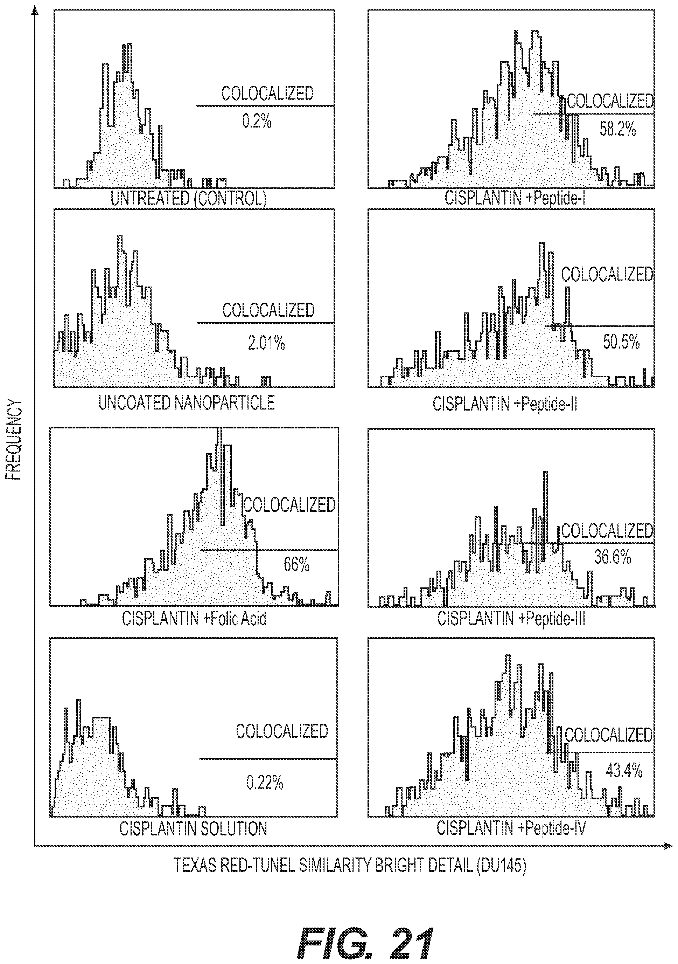

*PBM nanoparticle translocation to cell nuclei and TUNEL stain.* DU145 (prostate cancer cell line) cells were treated with cisplatin solution or cisplatin- and Texas Red-containing and cancer cell-binding peptides (I, II, III, and IV)- or folate-conjugated polycaprolactone-coated PBM nanoparticles, or uncoated nanoparticles. Cells are treated with cisplatin solution concentration(20uM) and nanoparticle with concentration (2uM)along with different peptides and folate, after treatments incubate the cells for 1 hour and than wash five times with PBS and again incubate cells for 48 hours in 37oC with 5% CO2, fixed and stained the cells with the TUNEL-FITC and Fluorescent DNA binding dye DRAQ5 and imaged on the Image stream system. FIG. 21 shows PBM nanoparticle translocation to cell nuclei and TUNEL stain. Amnis ImageStream Similarity Bright Detail analysis between TexasRed and TUNEL-FITC revealed co-localization of folic acid-, Peptide I-, Peptide II-, Peptide III-, and Peptide IV- coated PBM nanoparticles to the nuclei, which was not observed in uncoated particles.

## Claims

1. A pharmaceutical composition, comprising: planetary ball milled (PBM) nanoparticles comprising curcumin, wherein said PBM nanoparticles are coated with a cell targeting agent selected from the group consisting of folate, adenosine, purine, hormone, peptide ligand to a high affinity tumor receptor or a mixture thereof for use in a method of treating cancer in a subject in need thereof.

2. The pharmaceutical composition for use in the method of treatment of Claim 1, wherein said PBM nanoparticles further comprise one or more chemotherapeutic agents selected from the group consisting of platinum-based drugs, natural phenols, plant alkaloids and taxanes, other alkylating agents, tumor antibiotics and anthracyclines, topoisomerase inhibitors, antimetabolites, and miscellaneous antineoplastics.

3. The pharmaceutical composition for use in the method of treatment of Claim 1, wherein said PBM nanoparticles are coated with folate.

4. The pharmaceutical composition for use in the method of treatment of Claim 1, wherein the cancer is selected from the group consisting of carcinoma, sarcoma, lymphoma, leukemia, germ cell tumor, and blastoma.

5. The pharmaceutical composition for use in the method of treatment of Claim 1, wherein said PBM nanoparticles comprise a matrix core comprising polyethylene glycol (PEG) and another biodegradable polysaccharide selected from the group consisting of alginate, cellulose, collagen and starch.

6. The pharmaceutical composition for use in the method of treatment of Claim 5, wherein curcumin is entrapped within the matrix core.

7. The pharmaceutical composition for use in the method of treatment of Claim 1, wherein said PBM nanoparticles further comprise a release control coating comprising a biodegradable polymer selected from PEG, polycaprolactone (PCL) or a mixture thereof

8. The pharmaceutical composition for use in the method of treatment of Claim 1, wherein the cancer is a carcinoma.

9. The pharmaceutical composition for use in the method of treatment of Claim 1, wherein the cancer is breast cancer.

## Patentansprüche

1. Pharmazeutische Zusammensetzung, umfassend: Planeten-Kugelgemahlene (PBM) Nanopartikel, die Kurkumin umfassen, wobei die PBM Nanopartikel beschichtet sind mit einem Zielzellgerichteten Agens, ausgewählt aus der Gruppe bestehend aus Folat, Adenosin, Purin, Hormon, Peptidligand zu einem Tumor-Rezeptor mit hoher Affinität oder einer Mischung daraus, für die Nutzung bei einem Behandlungsverfahren von Krebs bei einem Subjekt, das dessen Bedarf.

2. Pharmazeutische Zusammensetzung für die Nutzung in dem Behandlungsverfahren nach Anspruch 1, wobei die PBM Nanopartikel zusätzlich ein oder mehrere Chemotherapeutika umfasst, ausgewählt aus der Gruppe bestehend aus platinbasierten Medikamenten, natürlichen Phenolen, pflanzlichen Alkaloiden und Taxanen, anderen alkylierenden Agenzien, Tumor-Antibiotika und Anthrazyklinen, Topoisomerasehemmern, Antimetaboliten und verschiedenen Antineoplastika.

3. Pharmazeutische Zusammensetzung für die Nutzung in dem Behandlungsverfahren nach Anspruch 1, wobei die PBM Nanopartikel mit Folat beschichtet sind.

4. Pharmazeutische Zusammensetzung für die Nutzung in dem Behandlungsverfahren nach Anspruch 1, wobei der Krebs ausgewählt wird aus der Gruppe bestehend aus Karzinomen, Sarkomen, Lymphomen, Leukämie, Keimzelltumor und Blastom.

5. Pharmazeutische Zusammensetzung für die Nutzung in dem Behandlungsverfahren nach Anspruch 1, wobei die PBM Nanopartikel einen Matrixkern umfassen, der Polyethylenglykol (PEG) und ein weiteres biologisch abbaubares Polysaccharid, ausgewählt aus der Gruppe bestehend aus Alginat, Zellulose, Kollagen und Stärke, umfasst.

6. Pharmazeutische Zusammensetzung für die Nutzung in dem Behandlungsverfahren nach Anspruch 5, wobei Kurkumin im Matrixkern eingeschlossen ist.

7. Pharmazeutische Zusammensetzung für die Nutzung in dem Behandlungsverfahren nach Anspruch 1, wobei die PBM Nanopartikel zusätzlich eine Freisetzungssteuerungsbeschichtung umfassen, die ein biologisch abbaubares Polymer, ausgewählt aus PEG, Polycaprolacton (PCL) oder einer Mischung daraus, umfasst.

8. Pharmazeutische Zusammensetzung für die Nutzung in dem Behandlungsverfahren nach Anspruch 1, wobei der Krebs ein Karzinom ist.

9. Pharmazeutische Zusammensetzung für die Nutzung in dem Behandlungsverfahren nach Anspruch 1, wobei der Krebs Brustkrebs ist.

## Revendications

1. Composition pharmaceutique comprenant : des nanoparticules broyées au broyeur planétaire à billes (PBM) comprenant de la curcumine, où lesdites particules PBM sont revêtues avec un agent de ciblage cellulaire choisi dans le groupe constitué d'un folate, de l'adénosine, de la purine, d'une hormone, d'un ligand peptidique à un récepteur de tumeur d'affinité élevée, ou d'un mélange de ceux-ci, pour une utilisation dans un procédé de traitement du cancer chez un sujet en ayant besoin.

2. Composition pharmaceutique pour une utilisation dans le procédé de traitement selon la revendication 1, dans laquelle lesdites nanoparticules PBM comprennent en outre un ou plusieurs agents chimio thérapeutiques choisis dans le groupe constitué de médicaments à base de platine, de phénols naturels, d'alcaloïdes et de taxanes de plantes, d'autres agents d'alkylation, d'antibiotiques pour tumeurs et d'anthracyclines, d'inhibiteurs de topo isomérase, d'anti métabolites et d'antinéoplasiques mélangés.

3. Composition pharmaceutique pour une utilisation dans le procédé de traitement selon la revendication 1, dans laquelle lesdites nanoparticules PBM sont revêtues de folate.

4. Composition pharmaceutique pour une utilisation dans le procédé de traitement selon la revendication 1, dans laquelle le cancer est sélectionné dans le groupe constitué du carcinome, du sarcome, du lymphome, de la leucémie, d'une tumeur de cellule germinale, et du blastome.

5. Composition pharmaceutique pour une utilisation dans le procédé de traitement selon la revendication 1, dans laquelle lesdites nanoparticules PBM comprennent un cœur de matrice comprenant du polyéthylène glycol (PEG) et un autre polysaccharide biodégradable choisi dans le groupe constitué d'alginate, de cellulose, de collagène et d'amidon.

6. Composition pharmaceutique pour une utilisation dans le procédé de traitement selon la revendication 5, dans laquelle la curcumine est piégée à l'intérieur du cœur de matrice.

7. Composition pharmaceutique pour une utilisation dans le procédé de traitement selon la revendication 1, dans laquelle lesdites nanoparticules PBM comprennent en outre un revêtement à libération contrôlée comprenant un polymère biodégradable choisi parmi le PEG, la polycaprolactone (PCL), ou un mélange de ceux-ci.

8. Composition pharmaceutique pour une utilisation dans le procédé de traitement selon la revendication 1, dans laquelle le cancer est un carcinome.

9. Composition pharmaceutique pour une utilisation dans le procédé de traitement selon la revendication 1, dans laquelle le cancer est le cancer du sein.
